(19) Europäisches Patentamt
European Patent Office
Office européen des brevets

(11) EP 1 043 403 A1

(12) EUROPEAN PATENT APPLICATION

(43) Date of publication:
**11.10.2000 Bulletin 2000/41**

(51) Int Cl.⁷: **C12Q 1/18**, C07K 14/245, C12Q 1/68

(21) Application number: **99107031.9**

(22) Date of filing: **09.04.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **GPC AG, Genome Pharmaceuticals Corporation**
**82152 Martinsried (DE)**

(72) Inventor: **The designation of the inventor has not yet been filed**

(74) Representative: **VOSSIUS & PARTNER**
**Siebertstrasse 4**
**81675 München (DE)**

Remarks:
The applicant has subsequently filed a sequence listing and declared, that it includes no new matter.

# (54) Novel method for identifying antibacterial compounds

(57) The present invention relates to a method for identifying an antagonist or inhibitor of the expression of a gene encoding a polypeptide essential for bacterial growth or survival as well as for an antagonist or inhibitor of said polypeptide. The invention further relates to a method for improved antagonists or inhibitors. The invention also provides an antagonist or inhibitor of the activity of said polypeptide. The invention is further related to a method for producing a therapeutic agent in a composition comprising said antagonist or inhibitor. Furthermore, the invention is related to the use of the polypeptide and the antagonist or inhibitor as well as to a method to identify a surrogate marker.

EP 1 043 403 A1

Annex to the application documents - subsequently filed sequences listing

SEQUENCE LISTING

```
<110> GPC Aktiengesellschaft

<120> Novel method for identifying antibacterial compounds

<130> D 1400 EP-a

<140>
<141>

<160> 50

<170> PatentIn Ver. 2.1

<210> 1
<211> 39
<212> DNA
<213> Artificial Sequence

<220>
<223> Description of Artificial Sequence: artificial
      sequence

<400> 1
atggttataa atttggagtg tgaaggttat tgcgtgtaa                             39


<210> 2
<211> 33
<212> DNA
<213> Artificial Sequence

<220>
<223> Description of Artificial Sequence: artificial
      sequence

<400> 2
gttataaatt tggagtgtga aggttattgc gtg                                   33


<210> 3
<211> 33
<212> DNA
<213> Artificial Sequence

<220>
<223> Description of Artificial Sequence: artificial
      sequence

<400> 3
cacgcaataa ccttcacact ccaaatttat aac                                   33


<210> 4
<211> 36
<212> DNA
<213> Artificial Sequence

<220>
<223> Description of Artificial Sequence: artificial
      sequence
```

<400> 4
gatcggatcc aaattccagt tagccatgat gcggtc 36

<210> 5
<211> 54
<212> DNA
<213> Artificial Sequence

<220>
<223> Description of Artificial Sequence: artificial sequence

<400> 5
cacgcaataa ccttcacact ccaaatttat aaccattata cacggacgct atgc 54

<210> 6
<211> 55
<212> DNA
<213> Artificial Sequence

<220>
<223> Description of Artificial Sequence: artificial sequence

<400> 6
gttataaatt tggagtgtga aggttattgc gtgacggatt aattttgttt ctctt 55

<210> 7
<211> 37
<212> DNA
<213> Artificial Sequence

<220>
<223> Description of Artificial Sequence: artificial sequence

<400> 7
gatcgtcgac gcgctcgata tcaccgatga acaaccg 37

<210> 8
<211> 25
<212> DNA
<213> Artificial Sequence

<220>
<223> Description of Artificial Sequence: artificial sequence

<400> 8
caatccgctg ctttatttct gtcag 25

<210> 9
<211> 25
<212> DNA
<213> Artificial Sequence

<220>
<223> Description of Artificial Sequence: artificial

sequence

<400> 9
ttataacgaa atcaacggga aacct 25

<210> 10
<211> 34
<212> DNA
<213> Artificial Sequence

<220>
<223> Description of Artificial Sequence: artificial
sequence

<400> 10
gatcggatcc ctctaaaaaa taaggaatta aagg 34

<210> 11
<211> 56
<212> DNA
<213> Artificial Sequence

<220>
<223> Description of Artificial Sequence: artificial
sequence

<400> 11
cacgcaataa ccttcacact ccaaatttat aaccatagga tacctaatta attaac 56

<210> 12
<211> 54
<212> DNA
<213> Artificial Sequence

<220>
<223> Description of Artificial Sequence: artificial
sequence

<400> 12
gttataaatt tggagtgtga aggttattgc gtgaagagcg ccatttccca ccgt 54

<210> 13
<211> 36
<212> DNA
<213> Artificial Sequence

<220>
<223> Description of Artificial Sequence: artificial
sequence

<400> 13
gatcgtcgac tcatattgct gataacccgc tgcggt 36

<210> 14
<211> 24
<212> DNA
<213> Artificial Sequence

<220>
<223> Description of Artificial Sequence: artificial
sequence

<400> 14
gttgacggcc aggccaacag tcat 24

<210> 15
<211> 22
<212> DNA
<213> Artificial Sequence

<220>
<223> Description of Artificial Sequence: artificial
sequence

<400> 15
ataaccctgg gcaatcgcct cg 22

<210> 16
<211> 480
<212> DNA
<213> Escherichia coli

<400> 16
atgcgaattg gacacggttt tgacgtacat gcctttggcg gtgaaggccc aattatcatt 60
ggtggcgtac gcattcctta cgaaaaagga ttgctggcgc attctgatgg cgacgtggcg 120
ctccatgcgt tgaccgatgc attgcttggc gcggcggcgc tgggggatat cggcaagctg 180
ttcccggata ccgatccggc atttaaaggt gccgatagcc gcgagctgct acgcgaagcc 240
tggcgtcgta ttcaggcgaa gggttatacc cttggcaacg tcgatgtcac tatcatcgct 300
caggcaccga agatgttgcc gcacattcca caaatgcgcg tgtttattgc cgaagatctc 360
ggctgccata tggatgatgt taacgtgaaa gccactacta cggaaaaact gggatttacc 420
ggacgtgggg aagggattgc ctgtgaagcg gtggcgctac tcattaaggc aacaaaatga 480

<210> 17
<211> 537
<212> DNA
<213> Escherichia coli

<400> 17
atgcgccgcg cttttataac cggagttttc tttttgtctg aagtcgaatt tagccacgaa 60
tactggatgc gtcacgcgct gacgctggcg aaacgtgcct gggatgagcg ggaagtgccg 120
gtcggcgcgg tattagtgca taacaatcgg gtaatcggcg aaggctggaa ccgcccgatt 180
ggtcgccatg atcccaccgc acatgcagaa atcatggccc tgcggcaggg tggtctggtg 240
atgcaaaatt atcgtctgat cgacgccacg ttgtatgtca cgcttgaacc atgtgtaatg 300
tgtgccggag cgatgatcca cagtcgcatt ggtcgcgtgg tctttggtgc gcgtgacgcg 360
aaaactggcg ctgcgggatc tttaatggat gtgctgcatc atccgggtat gaatcaccga 420
gtggaaatta cggaaggaat actggcggat gagtgcgcgg cgttgctcag tgacttcttt 480
cgcatgcgcc gccaggaaat taaagcgcag aaaaaagcgc aatcctcgac ggattaa 537

<210> 18
<211> 621
<212> DNA
<213> Escherichia coli

<400> 18
atgaggtata tagttgcctt aacgggaggc attggcagtg gcaagagtac cgttgccaat 60
gcgtttgctg atctcggaat taacgtcatt gatgccgata ttattgcgcg tcaggtggtt 120
gaaccaggtg cacctgcgct acatgccatt gctgatcact ttggcgctaa catgattgct 180

gctgatggaa cattgcagcg ccgggccttg cgcgagcgga tcttcgccaa cccggaagag 240
aaaaactggc ttaacgccct gctgcatccg ctgattcagc aagagacgca acaccagatc 300
cagcaagcta cttcccccta tgtactgtgg gttgtgccat tgctggtaga aaactcactg 360
tataaaaaag cgaatcgagt gcttgtggtg gatgtcagcc cagaaacgca acttaagcgc 420
accatgcagc gcgatgatgt aactcgcgag catgtcgaac aaatccttgc tgctcaggca 480
acgcgcgaag cccgccttgc cgtggcagat gacgtcattg ataataacgg cgcaccggat 540
gctatcgcat cggatgttgc ccgcctgcac gcacactatt tgcagcttgc gtcgcagttt 600
gtctcacagg aaaaaccgta a 621

<210> 19
<211> 852
<212> DNA
<213> Escherichia coli

<400> 19
atgcggacac agtggccctc tccggcaaaa cttaatctgt ttttatacat taccggtcag 60
cgtgcggatg gttaccacac gctgcaaacg ctgtttcagt ttcttgatta cggcgacacc 120
atcagcattg agcttcgtga cgatggggat attcgtctgt taacgcccgt tgaaggcgtg 180
gaacatgaag ataacctgat cgttcgcgca gcgcgattgt tgatgaaaac tgcggcagac 240
agcgggcgtc ttccgacggg aagcggtgcg aatatcagca ttgacaagcg tttgccgatg 300
ggcggcggtc tcggcggtgg ttcatccaat gccgcgacgg tcctggtggc attaaatcat 360
ctctggcaat gcgggctaag catggatgag ctggcggaaa tggggctgac gctgggcgca 420
gatgttcctg tctttgttcg gggcatgcc gcgtttgccg aaggcgttgg tgaaatacta 480
acgccggtgg atccgccaga gaagtggtat ctggtggcgc accctggtgt aagtattccg 540
actccggtga tttttaaaga tcctgaactc ccgcgcaata cgccaaaaag gtcaatagaa 600
acgttgctaa aatgtgaatt cagcaatgat tgcgaggtta tcgcaagaaa acgttttcgc 660
gaggttgatg cggtgctttc ctggctgtta gaatacgccc cgtcgcgcct gactgggaca 720
ggggcctgtg tctttgctga atttgataca gagtctgaag cccgccaggt gctagagcaa 780
gccccggaat ggctcaatgg ctttgtggcg aaaggcgcta atctttcccc attgcacaga 840
gccatgcttt aa 852

<210> 20
<211> 696
<212> DNA
<213> Escherichia coli

<400> 20
atgcgacttg ataaatttat cgcacagcaa ctcggcgtta gccgtgctat tgccgggcgt 60
gaaatccgcg gcaatcgtgt caccgtcgat ggcgaaatcg tccgtaatgc agcgttcaaa 120
ctgcttcctg aacatgatgt cgcttacgat ggcaacccgc tggcgcagca acacggtcca 180
cgttacttca tgctcaataa gcctcagggc tatgtttgct ccacggacga ccctgatcac 240
ccaacggtgc tctattttct tgatgaaccg gtagcgtgga aactgcatgc ggcggggcgg 300
ttggatattg ataccaccgg tctggtgctg atgactgatg atggtcagtg gtcgcaccgc 360
attacttctc cgcgccatca ttgcgagaag acctatctgg tgacactgga atcacctgta 420
gctgacgata cggcagagca atttgctaaa ggcgtgcagc tgcataacga aaaagatctc 480
actaagcctg cggtgctgga agtgattacc ccaacgcagg ttcgtctgac catcagcgaa 540
gggcgttatc atcaggtgaa acgcatgttc gccgccgtgg gtaaccacgt ggttgagctg 600
catcgtgaac gtattggcgg tattacgctg gatgctgatt tagcccccgg tgaatatcgt 660
ccgttaactg aagaagaaat tgccagcgtc gtctaa 696

<210> 21
<211> 885
<212> DNA
<213> Escherichia coli

<400> 21
atgattatgc cgcaacatga ccaattacat cgctatctgt ttgaaaactt tgccgtgcgc 60
ggcgaactgg taaccgtttc ggaaaccctg caacagatcc ttgagaacca cgattatccg 120
cagcccgtta aaaacgtgct ggcagaactg ctggttgcga ccagcctgtt aaccgctacg 180
ctgaagtttg atggtgatat caccgtacag ctgcagggcg acggtccgat gaatctggcg 240

gttattaacg gtaacaataa ccagcagatg cgcggtgtgg cgcgcgtgca gggcgaaatt 300
ccagaaaatg ccgacctgaa aacgctggtc ggcaatggtt acgtggtgat caccattacc 360
ccgagcgaag gcgaacgcta tcagggcgta gttggtctgg aaggtgatac cctggcggcc 420
tgcctggaag attactttat gcgttctgaa cagctgccga cgcgcctgtt tattcgcacc 480
ggcgacgtag acggcaaacc ggctgcaggc ggtatgttgt tgcaggtaat gcctgcgcaa 540
aatgcccagc aggacgactt tgaccacctg gcgacgctaa ccgaaaccat caaaaccgaa 600
gaactgctga ccttaccggc aaacgaagtg ttgtggcgtt tgtatcacga agaagaggtg 660
acggtttacg atccgcagga tgtggagttc aaatgcacct gctcgcgtga acgttgcgcc 720
gatgcgctga aaacgctgcc tgatgaagaa gttgatagca tcctggcgga agatggcgaa 780
attgacatgc attgtgatta ctgcggtaac cactatctgt tcaatgcgat ggatattgct 840
gaaatccgca acaacgcgtc tccggcagat ccgcaagttc attaa 885

<210> 22
<211> 759
<212> DNA
<213> Escherichia coli

<400> 22
gtggggagac gacgcggatt tttaactatg cgtatccccc gcatttatca tcctgaacca 60
ctgaccagcc attctcacat cgcgctttgc gaagatgccg ccaaccatat cgggcgcgta 120
ctgcgcatgg ggccggggca ggcgttgcaa ttgtttgacg gtagcaacca ggtctttgac 180
gccgaaatta ccagcgccag caaaaaaagc gtggaagtga aggtgctgga aggccagatc 240
gacgatcgcg aatctccgct gcatattcac ctcggtcagg tgatgtcgcg tggtgaaaaa 300
atggaattta ctatccagaa atcgatcgaa ctcggtgtaa gcctcattac gccacttttt 360
tctgagcgct gcggcgttaa actggatagt gaacgtctga acaagaagct tcagcagtgg 420
cagaagattg caattgctgc ctgtgagcag tgtggtcgta accgggtgcc ggaaatccgt 480
ccagcgatgg atctggaagc ctggtgtgca gagcaggatg aaggactgaa actgaatctt 540
cacccgcgcg ccagtaacag catcaatacg ttgccgttac cggttgaacg cgtccgcctg 600
ctgattggcc cggaaggcgg tttatcggca gatgaaattg ccatgactgc ccgctatcaa 660
tttactgata tcctgttggg acctcgcgtt ttgcgtacag agacaactgc gctcaccgcc 720
attaccgcgc tacaagtacg atttggcgat ttgggctaa 759

<210> 23
<211> 462
<212> DNA
<213> Escherichia coli

<400> 23
atgatgaatc gagtaattcc gctccctgat gagcaggcaa cattagacct gggcgagcgg 60
gtagcgaaag cctgcgatgg cgcaaccgta atctatctgt atggcgattt aggcgcaggt 120
aaaaccacct ttagccgggg ctttttacag gctctgggtc atcagggtaa tgtcaaaagc 180
cccacttata cgctggtcga accctatacg ctcgacaact taatggtcta tcactttgat 240
ttgtaccgcc ttgccgatcc cgaggagctg gagtttatgg ggatccgcga ttattttgcc 300
aacgatgcca tctgcctggt ggagtggcca caacaaggta caggtgttct tcctgacccg 360
gatgtcgaaa tacacattga ttatcaggca caaggccgtg aggcgcgcgt gagtgcggtt 420
tcctctgcgg gtgaattgtt gctggcgcgt ttagccggtt aa 462

<210> 24
<211> 987
<212> DNA
<213> Escherichia coli

<400> 24
atgaaattac gctctgtaac ctacgcatta ttcattgctg gcctggctgc attcagcaca 60
tcttctctgg cggcacaatc tttacgtttc ggttatgaaa catcacaaac cgactcgcaa 120
catattgcgg cgaaaaaatt caatgattta ttgcaggaga gaaccaaagg cgagctgaaa 180
ttaaaactgt tcccggacag cactctcggt aacgcgcagg cgatgatcag cggcgtacgt 240
ggcggcacca tcgatatgga aatgtccggc tcgaataact ttgccgggtt atcaccagtg 300
atgaacttgc ttgatgtccc tttcctgttc cgcgataccg ctcacgcgca taaaacgctc 360
gacggcaaag tcggtgatga tctgaaagcc tcacttgaag gtaaaggact gaaagtactg 420

gcctactggg aaaacggctg gcgcgatgtc accaactcgc gcgcaccggt taaaaccccc 480
gccgacctga aagggctgaa aatccgcacc aacaatagcc cgatgaatat cgccgcattc 540
aaagtctttg gcgctaaccc gatcccgatg ccgtttgccg aagtctatac cgggctggaa 600
acccgcacta tcgacgctca ggaacacccg atcaacgtcg tctggtcagc aaaatttttc 660
gaagtgcaga agttcctttc tctgacgcac cacgcctatt ccccgcttct ggtggtgatc 720
aacaaagcga agtttgatgg cttaagtccg gagttccagc aggcgctagt ttcatctgca 780
caagaagcgg gtaactatca gcgcaaactg gttgctgaag atcagcaaaa aatcatcgac 840
ggcatgaaag aagcgggcgt ggaagtcatc accgatctcg accgcaaagc ctttagcgac 900
gcactgggga atcaggttcg cgacatgttt gttaaagatg tgccgcaggg agctgatctg 960
ctgaaagccg tggatgaggt gcaataa 987

<210> 25
<211> 573
<212> DNA
<213> Escherichia coli

<400> 25
gtgaataata acctgcaaag agacgctatc gcagctgcga tagatgttct caatgaagaa 60
cgtgtcatcg cctatccaac ggaagccgtt ttcggtgttg ggtgcgatcc tgatagcgaa 120
acagcagtga tgcgactgtt ggagttaaaa cagcgtccgg ttgataaggg gctgatttta 180
atcgcagcaa attacgagca gcttaaaccc tatattgatg acaccatgtt gactgacgtg 240
cagcgtgaaa ccattttttc ccgctggcca ggtcctgtca cctttgtctt tcccgcgcct 300
gcgacaacac cgcgctggtt gacgggccgc tttgattcgc ttgctgtacg agtcaccgac 360
catccgttgg tggttgcttt gtgccaggct tatggtaaac cgctggtttc taccagtgcc 420
aacttgagtg gattgccacc ttgtcgaaca gtagacgaag ttcgcgcaca atttggcgcg 480
gcgttcccgg ttgtgcctgg tgaaacgggg ggcgtttaa atccttcaga aatccgcgat 540
gccctgacgg gtgaactgtt tcgacagggg taa 573

<210> 26
<211> 459
<212> DNA
<213> Escherichia coli

<400> 26
gtgggcttgt ccacattaga gcaaaaatta acagagatga ttactgcgcc agttgaggcc 60
ctgggttttg aactggttgg catcgaattt attcgcggtc gcacatccac actgcgcatc 120
tatattgata gtgaagatgg catcaatgtt gatgattgtg ctgatgtgag ccaccaggta 180
agtgctgtgc tggatgttga agatcccatc accgttgctt ataacctgga agtctcctca 240
ccgggtctcg atcgccccact gttcacggct gaacactacg cccgttttgt cggagaagag 300
gtgactctgg ttctccgtat ggcggtacaa aaccgtcgta aatggcaggg cgttatcaaa 360
gcggtagacg gtgaaatgat cacagttacc gtcgaaggta aagatgaagt gttcgcgctg 420
agtaatatcc agaaggcgaa cctggttccc cactttttaa 459

<210> 27
<211> 711
<212> DNA
<213> Escherichia coli

<400> 27
atggcaacca ctcatttgga tgtttgcgcc gtggttccgg cggccggatt tggccgtcga 60
atgcaaacgg aatgtcctaa gcaatatctc tcaatcggta atcaaaccat tcttgaacac 120
tcggtgcatg cgctgctggc gcatccccgg gtgaaacgtg tcgtcattgc cataagtcct 180
ggcgatagcc gttttgcaca acttcctctg gcgaatcatc cgcaaatcac cgttgtagat 240
ggcggtgatg agcgtgccga ttccgtgctg gcaggtctga aagccgctgg cgacgcgcag 300
tgggtattgg tgcatgacgc cgctcgtcct tgtttgcatc aggatgacct cgcgcgattg 360
ttggcgttga gcgaaaccag ccgcacgggg gggatcctcg ccgcaccagt gcgcgatact 420
atgaaacgtg ccgaaccggg caaaaatgcc attgctcata ccgttgatcg caacggctta 480
tggcacgcgc tgacgccgca atttttccct cgtgagctgt tacatgactg tctgacgcgc 540
gctctaaatg aaggcgcgac tattaccgac gaagcctcgg cgctggaata ttgcggattc 600
catcctcagt tggtcgaagg ccgtgcggat aacattaaag tcacgcgccc ggaagatttg 660

gcactggccg agttttacct cacccgaacc atccatcagg agaatacata a 711

<210> 28
<211> 468
<212> DNA
<213> Escherichia coli

<400> 28
atgagtcagg tgatcctcga tttacaactg gcatgtgaag ataattccgg gttaccggaa 60
gagagccagt ttcagacatg gctgaatgcg gtgatcccgc agtttcagga agaatcggaa 120
gtgacgattc gcgtggtcga taccgccgaa agccacagtc tgaatctgac ctatcgcggt 180
aaggataagc cgaccaacgt gctctccttc ccgtttgaag tgccgcctgg catggaaatg 240
tcgctactgg gcgatctggt tatctgccgt caggtggttg agaaggaagc tcaggagcaa 300
ggcaaaccac tggaggcgca ctgggcgcat atggtggtgc acggcagtct gcatttgtta 360
ggttacgatc acatcgaaga tgacgaagca gaagaaatgg aagccctcga aacagagatt 420
atgcttgctc tgggctatga ggatccgtac attgccgaga aagaataa 468

<210> 29
<211> 1119
<212> DNA
<213> Escherichia coli

<400> 29
atgcataacc aggctccaat tcaacgtaga aaatcaacac gtatttacgt tgggaatgtg 60
ccgattggcg atggtgctcc catcgccgta cagtccatga ccaatacgcg tacgacagac 120
gtcgaagcaa cggtcaatca aatcaaggcg ctggaacgcg ttggcgctga tatcgtccgt 180
gtatccgtac cgacgatgga cgcggcagaa gcgttcaaac tcatcaaaca gcaggttaac 240
gtgccgctgg tggctgacat ccacttcgac tatcgcattg cgctgaaagt agcggaatac 300
ggcgtcgatt gtctgcgtat taaccctggc aatatcggta atgaagagcg tattcgcatg 360
gtggttgact gtgcgcgcga taaaaacatt ccgatccgta ttggcgttaa cgccggatcg 420
ctggaaaaag atctgcaaga aaagtatggc gaaccgacgc cgcaggcgtt gctggaatct 480
gccatgcgtc atgttgatca tctcgatcgc ctgaacttcg atcagttcaa agtcagcgtg 540
aaagcgtctg acgtcttcct cgctgttgag tcttatcgtt tgctggcaaa acagatcgat 600
cagccgttgc atctggggat caccgaagcc ggtggtgcgc gcagcggggc agtaaaatcc 660
gccattggtt taggtctgct gctgtctgaa ggcatcggcg acacgctgcg cgtatcgctg 720
gcggccgatc cggtcgaaga gatcaaagtc ggtttcgata ttttgaaatc gctgcgtatc 780
cgttcgcgag ggatcaactt catcgcctgc ccgacctgtt cgcgtcagga atttgatgtt 840
atcggtacgg ttaacgcgct ggagcaacgc ctggaagata tcatcactcc gatggacgtt 900
tcgattatcg gctgcgtggt gaatggccca ggtgaggcgc tggtttctac actcggcgtc 960
accggcggca acaagaaaag cggcctctat gaagatggcg tgcgcaaaga ccgtctggac 1020
aacaacgata tgatcgacca gctggaagca cgcattcgtg cgaaagccag tcagctggac 1080
gaagcgcgtc gaattgacgt tcagcaggtt gaaaaataa 1119

<210> 30
<211> 480
<212> DNA
<213> Escherichia coli

<400> 30
atgcaaaaac gggcgattta tccgggtact ttcgatccca ttaccaatgg tcatatcgat 60
atcgtgacgc gcgccacgca gatgttcgat cacgttattc tggcgattgc cgccagcccc 120
agtaaaaaac cgatgtttac cctggaagag cgtgtggcac tggcacagca ggcaaccgcg 180
catctgggga acgtggaagt ggtcgggttt agtgatttaa tggcgaactt cgcccgtaat 240
caacacgcta cggtgctgat tcgtggcctg cgtgcggtgg cagattttga atatgaaatg 300
cagctggcgc atatgaatcg ccacttaatg ccggaactgg aaagtgtgtt tctgatgccg 360
tcgaaagagt ggtcgtttat ctcttcatcg ttggtgaaag aggtggcgcg ccatcagggc 420
gatgtcaccc atttcctgcc ggagaatgtc catcaggcgc tgatggcgaa gttagcgtag 480

<210> 31

<211> 699
<212> DNA
<213> Escherichia coli

<400> 31
atgaaaatcg gcatcattgg tgcaatggaa gaagaagtta cgctgctgcg tgacaaaatc 60
gaaaaccgtc aaactatcag tctcggcggt tgcgaaatct ataccggcca actgaatgga 120
accgaggttg cgcttctgaa atcgggcatc ggtaaagtcg ctgcggcgct gggtgccact 180
ttgctgttgg aacactgcaa gccagatgtg attattaaca ccggttctgc cggtggcctg 240
gcaccaacgt tgaaagtggg cgatatcgtt gtctcggacg aagcacgtta tcacgacgcg 300
gatgtcacgg catttggtta tgaatacggt cagttaccag gctgtccggc aggctttaaa 360
gctgacgata aactgatcgc tgccgctgag gcctgcattg ccgaactgaa tcttaacgct 420
gtacgtggcc tgattgttag cggcgacgct ttcatcaacg gttctgttgg tctggcgaaa 480
atccgccaca acttcccaca ggccattgct gtagagatgg aagcgacggc aatcgcccat 540
gtctgccaca atttcaacgt cccgtttgtt gtcgtacgcg ccatctccga cgtggccgat 600
caacagtctc atcttagctt cgatgagttc ctggctgttg ccgctaaaca gtccagcctg 660
atggttgagt cactggtgca gaaacttgca catggctaa 699

<210> 32
<211> 1344
<212> DNA
<213> Escherichia coli

<400> 32
gtgagcaatc tgtcgctcga tttttcggat aatacttttc aacctctggc cgcgcgtatg 60
cggccagaaa atttagcaca gtatatcggc cagcaacatt tgctggctgc ggggaagccg 120
ttgccgcgcg ctatcgaagc cgggcattta cattctatga tcctctgggg gccgccgggt 180
accggcaaaa caactctcgc tgaagtgatt gcccgctatg cgaacgctga tgtggaacgt 240
atttctgccg tcacctctgg cgtgaaagag attcgcgagg cgatcgagcg cgcccggcaa 300
aaccgcaatg caggtcgccg cactattctt tttgttgacg aagttcaccg tttcaacaaa 360
agccagcagg atgcatttct gccacatatt gaagacggca ccatcacttt tattggcgca 420
accactgaaa acccgtcgtt tgagcttaat tcggcactgc tttcccgtgc ccgtgtctat 480
ctgttgaaat ccctgagtac agaggatatt gagcaagtac taactcaggc gatggaagac 540
aaaacccgtg gctatggtgg tcaggatatt gttctgccag atgaaacacg acgcgccatt 600
gctgaactgg tgaatggcga cgcgcgccgg gcgttaaata cgctggaaat gatggcggat 660
atggccgaag tcgatgatag cggtaagcgg gtcctgaagc ctgaattact gaccgaaatc 720
gccggtgaac gtagcgcccg ctttgataac aaaggcgatc gcttttacga tctgatttcc 780
gcactgcata agtcggtacg tggtagcgca cccgatgcgg cgctgtactg gtatgcgcga 840
attattaccg ctggtggcga tccgttatat gtcgcgcgtc gctgtctggc gattgcgtct 900
gaagacgtcg gtaatgccga tccacgggcg atgcaggtgg caattgcggc ctgggattgc 960
tttactcgcg ttggcccggc ggaaggtgaa cgcgccattg ctcaggcgat tgtttacctg 1020
gcctgcgcgc caaaaagcaa cgctgtctac actgcgttta aagccgcgct ggccgatgct 1080
cgcgaacgcc cggattatga cgtgccggtt catttgcgta atgcgccgac gaaattaatg 1140
aaggaaatgg gctacgggca ggaatatcgt tacgctcatg atgaagcaaa cgcttatgct 1200
gccggtgagg tttacttccc gccggaaata gcacaaacac gctattattt cccgacaaac 1260
aggggccttg aaggcaagat tggcgaaaag ctcgcctggc tggctgaaca ggatcaaaat 1320
agccccataa aacgctaccg ttaa 1344

<210> 33
<211> 1911
<212> DNA
<213> Escherichia coli

<400> 33
gtgacggacg attttgcacc agacggtcag ctggcgaaag cgataccagg ctttaagccg 60
cgagaaccac agcgacagat ggcggtagcc gtcacccagg cgatagaaaa aggccagccg 120
ctggtggtgg aagcaggaac cggtacgggc aaaacctacg cttacctggc tcctgcgctg 180
cgggcgaaaa agaaagtcat tatctcgacc ggctcaaaag cgttgcagga tcagctctac 240
agccgcgatt tgccaacagt ctcaaaggca ttgaaatata cgggcaacgt ggcgctgctg 300
aaagggcgct caaactacct ctgcctcgaa cgtctcgaac agcaggcgct ggcgggggc 360
gatctgccgg tacaaatctt aagcgatgtg atcctgctgc gctcctggtc taatcaaaca 420

gtcgatggtg atatcagcac ctgcgtcagc gtggcggaag attcacaggc gtggccgctg 480
gtcaccagca ccaacgacaa ctgtcttggc agcgactgcc cgatgtataa agattgcttt 540
gtggtcaaag cacgtaaaaa agcgatggac gccgatgtgg tggtggtaaa ccatcatctc 600
tttctggcgg atatggtggt taaagagagt ggatttggcg aactgatccc ggaagcggac 660
gtcatgatct tcgacgaagc ccaccagcta ccggacattg ccagccagta ttttggtcag 720
tcactctcca gtcgacaact gctcgacctg gcaaaagaca tcaccatcgc ctaccgcacc 780
gaattaaaag acacccagca gttacaaaag tgcgctgatc gtcttgccca gagtgcgcag 840
gattttcgtc tgcaactcgg tgagccaggt tatcgcggta acctgcgtga gctgttagct 900
aatccgcaaa ttcagcgggc atttttactg ctcgatgaca ccctggaact ttgttatgac 960
gtggcgaaac tgtcactggg gcgttccgcc ttgctggatg cggcatttga gcgcgccacg 1020
ttgtatcgca cacggctgaa gcggctaaaa gagatcaatc agccgggcta cagctactgg 1080
tacgaatgca cttcgcgcca ttttactctg gctctcacgc cgctcagcgt ggcggataaa 1140
ttcaaagagt taatggcgca aaaacccggt agctggatct tcacctcagc aacgctgtcg 1200
gtgaacgacg atctgcatca tttcacctcg cggcttggca tcgaacaggc cgagtcgttg 1260
ctgttgccca gcccatttga ttacagccgc caggcgttac tctgtgtgct gcgcaatctg 1320
ccgcaaacca accagccagg ttctgctcgc cagttagcgg caatgctgcg accgatcatc 1380
gaagctaaca acggtcgttg ttttatgctt tgtacctcgc acgccatgat gcgcgatctg 1440
gccgagcagt tccgcgctac catgacgctt cctgtattgt tgcagggggga aaccagcaaa 1500
gggcaactgt tgcagcaatt tgtcagcgcc ggtaatgcgc ttcttgtggc aaccagcagt 1560
ttctgggaag gggtggacgt gcgtggcgat acattgtcat tggtaattat cgacaaattg 1620
ccgtttacct cgccggatga tccactgtta aaagcgcgca tggaagattg tcgtttgcgc 1680
ggtggcgacc cgttcgatga agtgcaacta ccagatgccg tcattactct caaacagggg 1740
gtagggcgac tgattcgcga cgccgacgat cgtggcgtgc tggtgatttg tgacaatcgg 1800
ctggtgatgc gtccttacgg cgcgacgttt ctcgccagtc tgccgcccgc gccacgcacc 1860
cgtgacattg cccgtgcggt tcgtttcctt gcgataccat cctccaggta a 1911

<210> 34
<211> 414
<212> DNA
<213> Escherichia coli

<400> 34
atggctaata aaccttcggc agaagaactg aaaaaaaatt tgtccgagat gcagttttac 60
gtgacgcaga atcatgggac agaaccgcca tttacgggtc gtttactgca taacaagcgt 120
gacggcgtat atcactgttt gatctgcgat gccccgctgt ttcattccca aaccaagtat 180
gattccggct gtggctggcc cagtttctac gaaccggtaa gtgaagaatc cattcgttat 240
atcaaagact tgtcacatgg aatgcagcgc atagaaattc gttgcggtaa ctgtgatgcc 300
catctggggc atgtcttccc cgacgggccg cagccaacgg gcgaacgtta ttgtgttaac 360
tctgcctctt tacgctttac cgatggcgaa aacggcgaag aaatcaacgg ttga 414

<210> 35
<211> 1968
<212> DNA
<213> Escherichia coli

<400> 35
atgaccattg agaaaatttt caccccgcag gacgacgcgt tttatgcggt gatcacccac 60
gcggcggggc cgcagggcgc tctgccgctg accccgcaga tgctgatgga atctcccagc 120
ggcaacctgt tcggcatgac gcagaacgcc gggatgggct gggacgccaa caagctcacc 180
ggcaaagagg tgctgattat cggcactcag ggcggcatcc gcgccggaga cggacgccca 240
atcgcgctgg gctaccacac cgggcattgg gagatcggca tgcagatgca ggcggcggcg 300
aaggagatca cccgcaatgg cgggatcccg ttcgcggcct tcgtcagcga tccgtgcgac 360
gggcgctcgc agggcacgca cggtatgttc gattccctgc cgtaccgcaa cgacgcggcg 420
atcgtgtttc gccgcctgat ccgctccctg ccgacgcggc gggcggtgat cggcgtagcg 480
acctgcgata aagggctgcc cgccaccatg attgcgctgg ccgcgatgca cgacctgccg 540
actattctgg tgccgggcgg ggcgacgctg ccgccgaccg tcggggaaga cgcgggcaag 600
gtgcagacca tcggcgcgcg tttcgccaac cacgaactct ccctgcagga ggccgccgaa 660
ctgggctgtc gcgcctgcgc ctcgccgggc ggcgggtgtc agttcctcgg cacggcgggc 720
acctcgcagg tggtcgcgga ggcgctgggt ctggcgctgc cgcactccgc gctggcgccg 780
tccgggcagg cggtgtggct ggagatcgcc cgccagtcgg cgcgcgcggt cagcgagctg 840
gatagccgcg gcatcaccac gcgggatatc ctctccgata aagccatcga aaacgcgatg 900

gtgatccacg cggcgttcgg cggctccacc aatttactgc tgcacattcc ggccatcgcc 960
cacgcggcgg gctgcacgat cccggacgtt gagcactgga cgcgcatcaa ccgtaaagtg 1020
ccgcgtctgg tgagcgtgct gcccaacggc ccggactatc acccgaccgt gcgcgccttc 1080
ctcgcgggcg gcgtgccgga ggtgatgctc cacctgcgcg acctcggcct gctgcatctg 1140
gacgccatga ccgtgaccgg ccagacggtg ggcgagaacc ttgaatggtg gcaggcgtcc 1200
gagcgccggg cgcgcttccg ccagtgcctg cgcgagcagg acggcgtaga gccggatgac 1260
gtgatcctgc cgccggagaa ggcaaaagcg aaagggctga cctcgacggt ctgcttcccg 1320
acgggcaaca tcgctccgga aggttcggtg atcaaggcca cggcgatcga cccgtcggtg 1380
gtgggcgaag atggcgtata ccaccacacc ggccgggtgc gggtgtttgt ctcggaagcg 1440
caggcgatca aggcgatcaa gcgggaagag attgtgcagg gcgatatcat ggtggtgatc 1500
ggcggcgggc cgtccggcac cggcatggaa gagacctacc agctcacctc cgcgctaaag 1560
catatctcgt ggggcaagac ggtgtcgctc atcaccgatg cgcgcttctc gggcgtgtcg 1620
acgggcgcct gcttcggcca cgtgtcgccg gaggcgctgg cgggcgggcc gattggcaag 1680
ctgcgcgata acgacatcat cgagattgcc gtggatcgtc tgacgttaac tggcagcgtg 1740
aacttcatcg gcaccgcgga caacccgctg acgccggaag agggcgcgcg cgagctggcg 1800
cggcggcaga cgcacccgga cctgcacgcc cacgactttt tgccggacga cacccggctg 1860
tgggcggcac tgcagtcggt gagcggcggc acctggaaag gctgtattta tgacaccgat 1920
aaaattatcg aggtaattaa cgccggtaaa aaagcgctcg gaatttaa 1968

<210> 36
<211> 717
<212> DNA
<213> Escherichia coli

<400> 36
gtgggacgta aatgggccaa tattgttgct aaaaaaacgg ctaaagacgg tgcaacgtct 60
aaaatttatg caaaattcgg tgtagaaatc tatgctgctg ctaaacaagg tgaacccgat 120
ccagaattaa acacatcttt aaaattcgtt attgaacgtg caaagcaggc acaagttcca 180
aagcacgtta ttgataaagc aattgataaa gccaaaggcg gcggagatga aacgttcgtg 240
cagggacgtt atgaaggctt tggtcctaat ggctcaatga ttatcgccga gacattgact 300
tcaaatgtta accgtacgat tgctaacgtt cgcacaattt tcaataaaaa aggcggcaat 360
atcggagcgg caggttctgt cagctatatg tttgacaata cgggtgtgat tgtatttaaa 420
gggacagacc ctgaccatat ttttgaaatt ttacttgaag ctgaagttga tgttcgtgat 480
gtgactgaag aagaaggtaa cattgttatt tatactgaac ctactgacct tcataaagga 540
atcgcggctc taaaagcagc tggaatcact gagttctcaa caacagaatt agaaatgatt 600
gctcaatctg aagttgagct ttccccagaa gatttagaaa tctttgaagg gcttgttgat 660
gcccttgaag atgacgacga tgtacaaaaa gtttatcata acgtcgcaaa tctctaa 717

<210> 37
<211> 258
<212> DNA
<213> Escherichia coli

<400> 37
atggcgccgc cactgtcgcc tggctcgcgg gtcctgatag ccctcattcg ggtctatcaa 60
cgcctgatta gtccgctact cgggccgcat tgtcgtttca ctccaacctg ttcaagctac 120
ggaattgagg cattgcgcag gtttggagtg ataaaaggca gttggttgac ggtgaaacgc 180
gtattaaaat gccacccttt acaccctggt ggtgacgatc ccgtcccgcc cggaccattt 240
gataccagag aacactaa 258

<210> 38
<211> 1023
<212> DNA
<213> Escherichia coli

<400> 38
atgaaaaaag tgttattgat aatcttgtta ttgctggtgg tactgggtat cgccgctggt 60
gtgggcgtct ggaaggttcg ccatcttgcc gacagcaaat tgcttatcaa agaagagacg 120
atatttaccc tgaagccagg gaccggacgt ctggcgctcg gtgaacagct ttatgccgat 180
aagatcatca atcgtccacg ggtttttcaa tggctgctgc gtatcgaacc ggatctttct 240

cactttaaag ccgggactta ccgctttaca ccgcagatga ccgtgcgcga gatgctgaaa 300
ttgctggaaa gcggtaaaga agcacagttc cctctgcgac tggtagaagg gatgcgtctg 360
agcgattacc tcaagcaatt gcgtgaggcc ccgtatatca agcatacgct gagcgatgat 420
aagtacgcca ccgtagcgca ggcacttgaa ctggaaaacc cggagtggat tgaaggttgg 480
ttctggccag acacctggat gtataccgcc aataccaccg atgtcgcgtt actcaagcga 540
gcgcacaaga aaatggtgaa agcggtcgat agcgcctggg aagggcgtgc ggacggtctg 600
ccttataaag ataaaaacca gttggtgacg atggcatcaa ttatcgaaaa agaaaccgcc 660
gttgccagtg aacgcgataa ggttgcctca gtatttatca accgtttacg cattggtatg 720
cgcctgcaga ccgacccgac cgtgatttac gggatgggag agcgttataa tggcaaactt 780
tctcgtgcag acctggaaac gccgacagcg tataacacct ataccattac cggtctgccg 840
ccaggtgcga tagcgacgcc gggggcggat tcgctgaagg ctgctgcgca tccggcaaaa 900
acgccgtatc tctattttgt ggccgatggt aaaggtggtc acacgtttaa taccaatctt 960
gccagtcata acaagtctgt gcaggattat ctgaaagtgc ttaaggaaaa aaatgcgcag 1020
taa 1023

<210> 39
<211> 873
<212> DNA
<213> Escherichia coli

<400> 39
atgctgcccg actcatcagt ccgtttaaat aaatacatca gcgaaagcgg aatttgctca 60
cgccgcgaag cggatcgcta tatcgagcaa ggcaatgtgt tccttaatgg caagcgagcc 120
accattggcg atcaggtgaa acccggcgac gttgtgaaag taaacggtca gttgattgaa 180
cctcgggaag ccgaagattt ggtacttatc gccctgaaca agcccgttgg tattgtaagc 240
accaccgaag atggcgagcg cgataacatt gtcgatttcg ttaaccacag caaacgcgtg 300
ttcccgattg gccgcctgga taaagactcc caggggctga ttttcctcac caatcacggc 360
gatctggtga ataagatcct gcgtgctggc aatgatcatg agaaagagta tctggtgacg 420
gtcgataaac cgattaccga ggagtttatt cgcggcatga gtgcgggggt gccaatcctc 480
gggacagtga ccaaaaagtg caaagttaaa aaagaagcgc cgtttgtctt ccgcattacc 540
ctggtgcagg ggctgaaccg tcagatccgg cgcatgtgcg agcatttcgg ctatgaagtg 600
aaaaagctgg aacgcacgcg catcatgaac gttagcttaa gcggcattcc gctgggggaa 660
tggcgcgatt taaccgacga tgagttaatc gacctcttta agctcattga aaattcctct 720
tccgaggtaa aacctaaagc gaaggccaaa ccgaaaacag cgggcatcaa acgtccagtc 780
gttaagatgg aaaaaacggc ggaaaaaggc ggtcgcccgg cgtccaacgg taagcgtttt 840
acctcgccgg ggcgtaaaaa gaagggggcgc tga 873

## Description

The present invention relates to a method for identifying an antagonist or inhibitor of the expression of a gene encoding a polypeptide essential for bacterial growth or survival as well as for an antagonist or inhibitor of said polypeptide. The invention further relates to a method for improved antagonists or inhibitors. The invention also provides an antagonist or inhibitor of the activity of said polypeptide. The invention is further related to a method for producing a composition comprising said antagonist or inhibitor. Furthermore, the invention is related to the use of the polypeptide and the antagonist or inhibitor as well as to a method to identify a surrogate marker.

Several documents are cited throughout the text of this specification. Each of the documents cited herein (including any manufacturer's specifications, instructions, etc.) are hereby incorporated by reference; however, there is no admission that any document cited is indeed prior art of the present invention.

Since the beginning of the 1980s, a new trend has been observed in the industrialized countries. On the one hand, resistances to antibiotics have increased, which make it difficult or even impossible to treat many of the disease-causing agents. On the other hand, new infectious diseases, which had been unknown up to now, arise, and old diseases return. For example, diphteria and tuberculosis are old epidemics and increasingly surmounting in many different parts of the world. Especially tuberculosis (TB), a chronic infectious disease that is generally caused by infection with *Mycobacterium tuberculosis,* is a disease of major concern. Each year, 8 to 10 million new cases of TB are described, and, causing more than three million deaths per year, TB is a major disease in developing countries as well as an increasing problem in developed areas of the world due to, for example, antibiotic resistance.
Additionally, *M. bovis* BCG vaccination has failed to protect against TB in several trials (WHO, Tech. Rep. Ser. (1980), 651, 1-15) for reasons that are not entirely clear (Fine, Tubercle 65 (1984), 137-153). It has been shown that the vaccine strain of M. bovis BCG only confers protection against the severe form of miliary tuberculosis in children (Fine, Lancet 346 (1995), 1339-1345). In contrast, its protective capacity against the most common form, pulmonary tuberculosis in adults, is low and highly variable (Colditz (1994), JAMA 271, 698).

The causes for this new trend are complex: mainly, the increasing number of antibiotic applications in medicine and agriculture often combined with an improper und uncontrolled use, helps to establish resistant organisms and generate the threat of bacterial infections resistant to all available therapies.
Conventional techniques of developing antibiotics, i.e. synthesis of candidate substances and screening for antibacterial substances, even though speeded up by several orders of magnitude by the use of combinatorial approaches in recent years (e.g. US5324483, US5545568), are still too inefficient as they involve multiple screening steps of hundreds or thousands of more or less randomly chosen substances for efficiency in combating various infectious agents.
Therefore, it is a major concern to fight the growing number of bacterial infections due to an increased frequency of multiple antibiotic resistances and to improve the available antibacterial therapies.

Thus, the technical problem underlying the present invention was to provide a method and means for the development of an additional effective antibacterial therapy of infected humans and animals that can be used for the treatment of a broad spectrum of bacterial infections or diseases or disorders related to bacterial infections. The solution to this technical problem is achieved by providing the embodiments characterized in the claims.

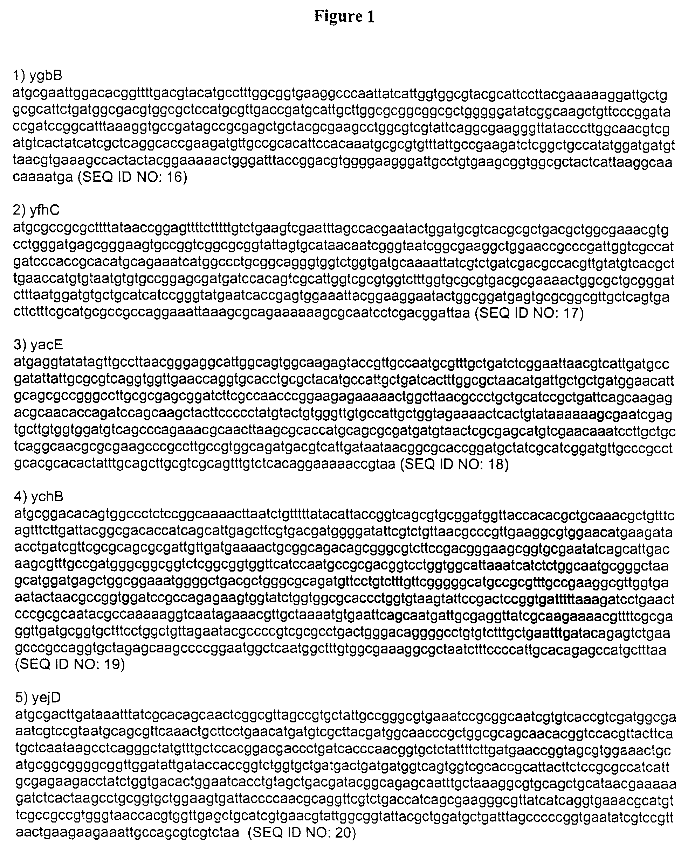

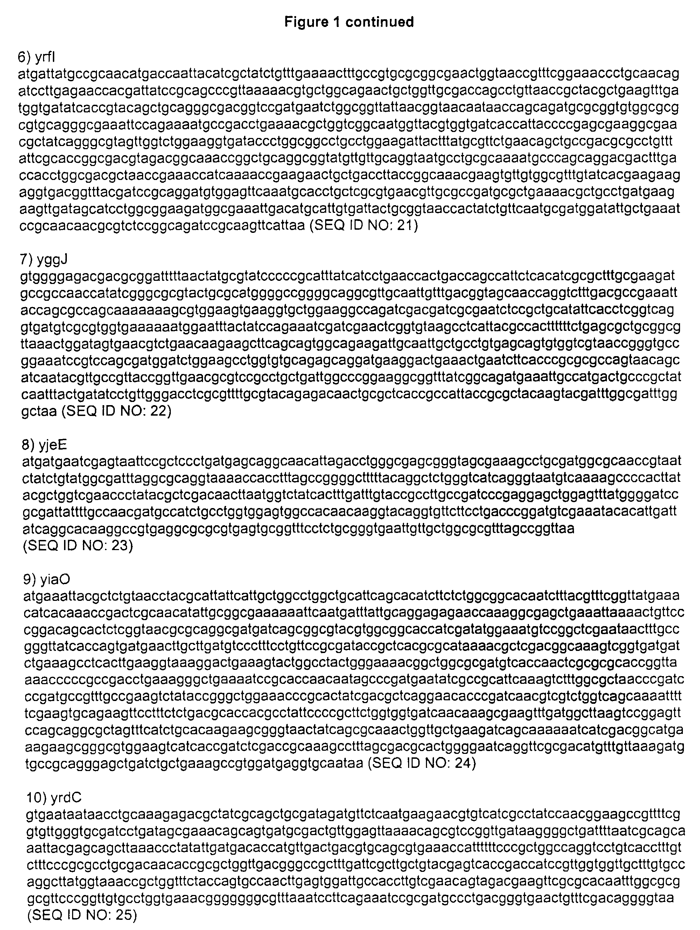

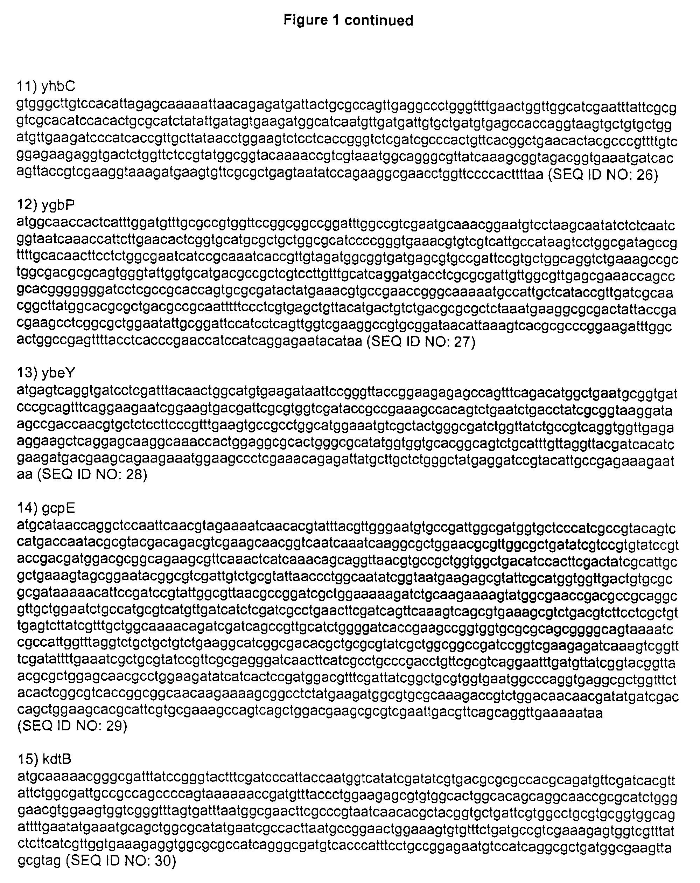

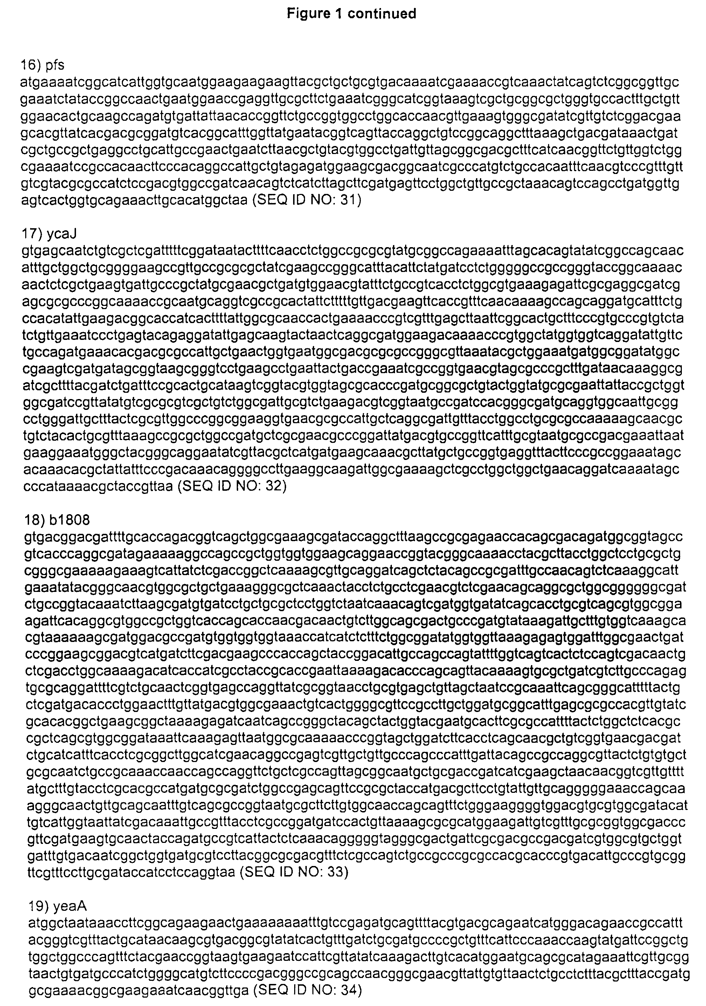

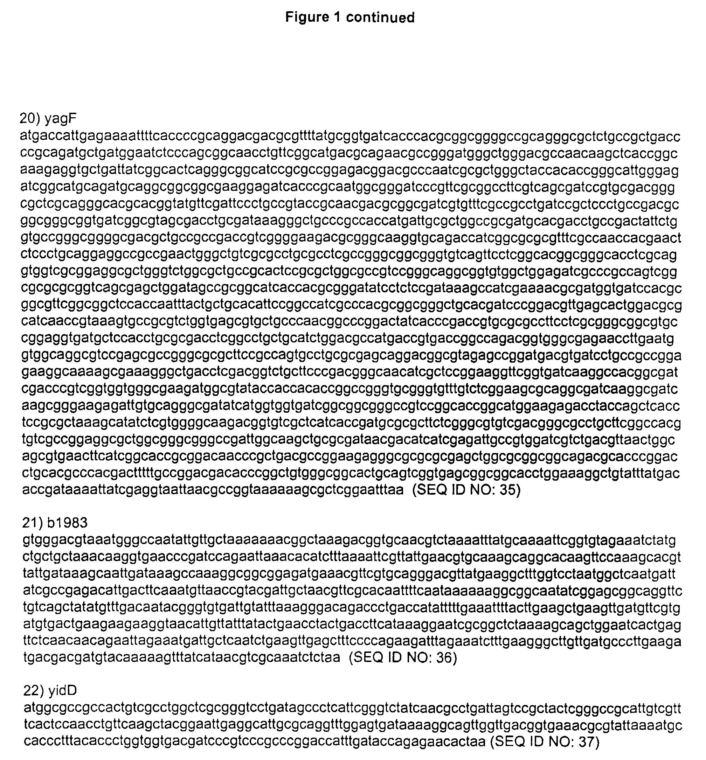

Accordingly, the present invention relates to a method for identifying an antagonist or inhibitor of the expression of a gene encoding a polypeptide essential for bacterial growth wherein said gene is selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or a fragment or derivative or ortholog thereof, said method comprising the steps of

(a) testing a candidate antagonist or inhibitor or a sample comprising a plurality of said candidate antagonists or inhibitors for the inhibition or reduction of transcription of said gene or a fragment or derivative thereof; or

(b) testing a candidate antagonist or inhibitor or a sample comprising a plurality of said candidate antagonists or inhibitors for the inhibition or reduction of translation of mRNA transcribed from said gene or a fragment or derivative thereof; and

(c) identifying an antagonist or inhibitor or a sample comprising a plurality of said candidate antagonists or inhibitors that tests positive in step (a) and/or (b).

The term "antagonist" or "inhibitor" as used herein means naturally occurring and synthetic compounds capable of counteracting or inhibiting an activity of a gene or gene product or interactions of the gene or gene product with other genes or gene products. Determining whether a compound is capable of inhibiting or counteracting specific gene expression can be done, for example, by Northern blot analysis, Western blot analysis or proteome analysis. It can further be done by monitoring the phenotypic characteristics of a bacterial cell contacted with the compounds and compare it to that of a wild-type cell. In an additional embodiment, said characteristics may be compared to that of a cell contacted with a compound which is either known to be capable or incapable of suppressing or activating the protein or gene, respectively, according to the invention. For example, the bacterial cell can be a transgenic cell and the phenotypic characteristics comprises a readout system. Further examples of determining whether a compound is capable of inhibiting or counteracting specific gene expression are described below.

The term "expression" means the production of a protein or nucleotide sequence in a cell. However, said term also includes expression of the protein in a cell-free system. It includes transcription into an RNA product, and/or translation into a polypeptide from a DNA encoding that product.

The term "transcription" as used herein means a DNA template dependent synthesis of a ribonucleic acid polymer encoding a polypeptide or a regulatory sequence. The term "translation" as used herein means the polymerization of a polypeptide that is encoded by an RNA molecule by a protein complex.

As used in accordance with the present invention, the term "fragment or derivative" denotes any variant the amino acid or nucleotide sequence of which deviates in its primary structure, e.g., in sequence composition or in length as well as to analogue components. For example, one or more amino acids of a polypeptide may be replaced in said fragment or derivative as long as the modified polypeptides remain functionally equivalent to their described counterparts. The term "fragment or derivative" further denotes compounds analog to an antagonist or inhibitor that should have a stabilized electronic configuration and molecular conformation that allows key functional groups to be presented to the mentioned polypeptide in substantially the same way as the antagonist and inhibitor. The variant of the polypeptide may be a naturally occurring allelic variant of the polypeptide or non-naturally occurring variants of those polynucleotides.

The term "orthologs" as used herein means homologous sequences in different species that evolved from a common ancestoral gene by speciation. Normally, orthologs retain the same function in the course of evolution. However, orthologous genes may or may not be responsible for a similar function (see, e.g., the glossary of the "Trends Guide to Bioinformatics", Trends Supplement 1998, Elsevier Science). Orthologous genes, nucleic acids or proteins comprise genes, nucleic acids or proteins which have one or more sequences or structural motifs in common. For example, the sequence motifs of proteins can comprise short, i.e. repetitive sequences or amino acid positions conserved in the primary structure and/or conserved in higher protein structures, e.g. secondary or tertiary structure. Orthologous nucleic acids or genes can comprise molecules having short stretches of one or more homologous (same or similar) sequences, for example protein binding boxes or structure forming boxes. Methods for the identification of a candidate ortholog of a gene or polypeptide described herein are known to those skilled in the art and are described for example in Sambrook et al. (1989), Molecular Cloning: A Laboratory Manual, Cold Spring Harbor Laboratory Press, New York, or Ausubel (1994), Current Protocols in Mol. Biol.. The person skilled in the art knows how to identify orthologous genes, nucleic acids or polypeptides by computer supported analysis (e.g. BLAST) of known sequences and its interpretation.

The terms "gene", "polynucleotide", "nucleic acid sequence", "nucleotide sequence", "DNA sequence" or "nucleic acid molecule" as used herein refer to polymeric forms of nucleotides of any length, either ribonucleotides or deoxyribonucleotides and only to the primary structure of the molecule. Thus, these terms include double- and single-stranded DNA, and RNA. They also include known types of modifications, for example, methylation, "caps" substitution of one or more of the naturally occurring nucleotides with an analog. Preferably, the DNA sequence of the invention comprises a coding sequence encoding at least the mature form of the above defined protein, i.e. the protein which is posttranslationally processed in its biologically active form, for example due to cleavage of leader or secretory sequences or a proprotein sequence or other natural proteolytic cleavage points.

The term "plurality of candidate antagonists or inhibitors" is to be understood as a plurality of substances which may or may not be identical.

Said antagonists or inhibitors or plurality of candidate antagonists or inhibitors may be chemically synthesized or microbiologically produced and/or comprised in, for example, samples, e.g., cell extracts from, e.g., plants, animals or microorganisms. Furthermore, said compound(s) may be known in the art but hitherto not known to be capable of suppressing or inhibiting said polypeptide. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994), in particular Chapter 17. The plurality of compounds may be, e.g., added to the reaction mixture, culture medium, injected into the cell or sprayed onto the plant.

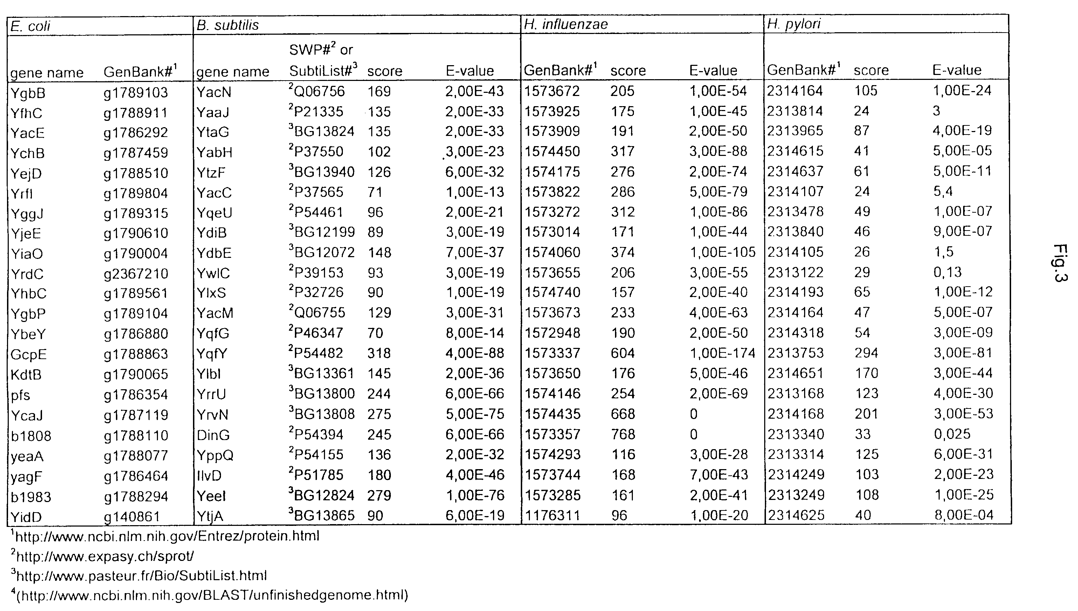

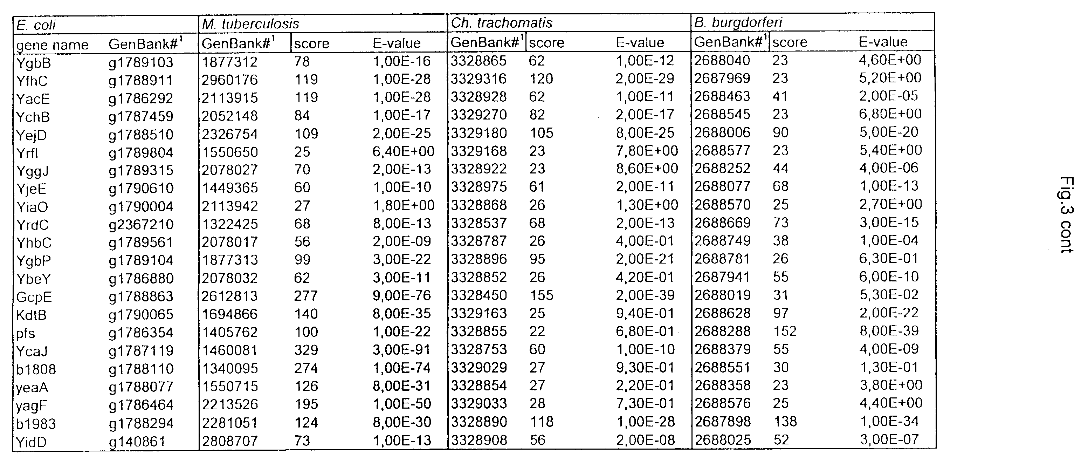

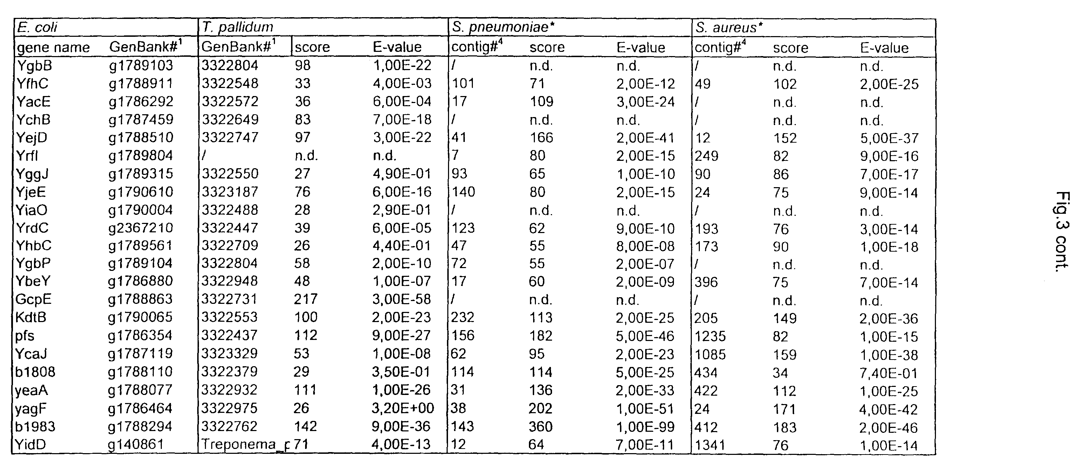

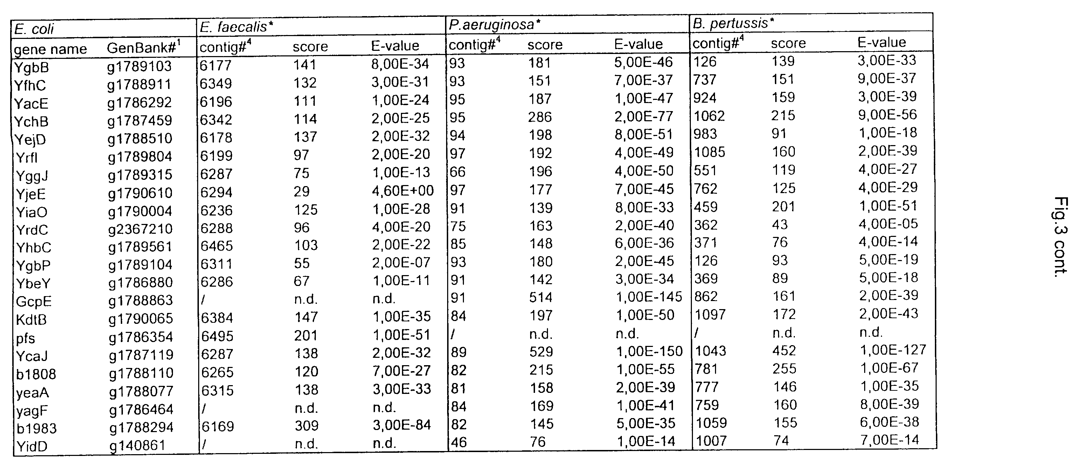

By combining computational processing of genomic information with microbial genetics, the inventors have been able to identify 22 E. coli essential genes and their respective orthologs (Fig. 3) that fulfill several criteria for being attractive antibacterial targets: hypothetical open reading frames, coding for essential functions (mutation is lethal for growth in rich media), broad conservation (orthologs are present in a wide range of bacteria including *H. influenza, S. pneumoniae, H. pylori,* and *B. burgdorferi)* (Fig. 3) and low toxicity potential in higher organisms (mostly no orthologs are identified in the simple eukaryote *S. cerevisiae).* Thus, an antagonist or inhibitor of the expression of such an essential gene or of its function provides the key for an antibacterial therapy. The inventors assume that said antagonist or inhibitor stops or reduces bacterial growth and/or mediates bacterial death.

Thus, the method of the present invention provides the options of development of new broad spectrum antibiotics against new pharmaceutical important targets. The findings of the present invention are particularly important in view of the drawbacks of the present forms of treatment of bacterial infections, diseases and disorders related to bacterial infections.

In line with the above, the present invention also relates to a method for testing a candidate antagonist or inhibitor of a polypeptide or mRNA essential for bacterial growth or survival encoded by a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD or a fragment, derivative or ortholog thereof comprising the steps of

(a) contacting a bacterial cell with candidate antagonist or inhibitor or a sample comprising a plurality of said candidate antagonists or inhibitors; and
(b) testing whether said contacting leads to cell growth inhibition and/or cell death.

In a further embodiment, the present invention relates to a method for testing a candidate antagonist or inhibitor of the function of a gene essential for bacterial growth or survival wherein said gene is selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD or a fragment, derivative or ortholog thereof, comprising the steps of

(a) contacting a bacterial cell comprising said gene with a candidate antagonist or inhibitor or a sample comprising a plurality of said candidate antagonists or inhibitors; and
(b) testing whether said contacting leads to cell growth inhibition and/or cell death.

Bacteria, for which was shown that a gene as mentioned above expressed is essential, can be used in a proliferation assay to identify both ligands and potential antagonists or inhibitors to said polypeptide encoded by said essential gene. For example, E. coli are grown in culture medium and incorporation of DNA precursors such as $^3$H-thymidine or 5-bromo-2'-deoxyuridine (BrdU) is monitored as a parameter for DNA synthesis and cellular proliferation. Cells which have incorporated BrdU into DNA can be detected using a monoclonal antibody against BrdU and measured by an enzyme or fluorochrome-conjugated second antibody. The reaction is quantitated by fluorimetry or by spectrophotometry. The ability of the compound to be screened to inhibit proliferation may then be quantified. Further methods to determine growth and proliferation of bacteria are well known in the art, for example in Drews, Mikrobiol. Praktikum, Berlin, 1976.

Preferably, the antagonist or inhibitor binds to the gene product, i.e. the RNA or polypeptide, specifically encoded by said gene.

For example, a candidate antagonist or inhibitor not known to be capable of binding to an polypeptide encoded by a essential gene as described above can be tested to bind thereto comprising contacting a bacterial cell comprising an isolated molecule encoding said polypeptide with a candidate antagonist or inhibitor under conditions permitting binding of ligands known to bind thereto, detecting the presence of any bound ligand, and thereby determining whether such candidate antagonist or inhibitor inhibits the binding of a ligand to a polypeptide as described above.

Proteins that bind to a polypeptide as described above and might inhibit or counteract to said polypeptide can be "captured" using the yeast two-hybrid system (Fields, Nature 340 (1989), 245-246). A modified version of the yeast two-hybrid system has been described by Roger Brent and his colleagues (Gyuris, Cell 75 (1993), 791-803; Zervos, Cell 72 (1993), 223-232). Briefly, a domain of the polypeptide is used as bait for binding compounds. Positives are then selected by their ability to grow on plates lacking leucine, and then further tested for their ability to turn blue on plates with X-gal, as previously described in great detail (Gyuris, supra; WO 95/31544). Once amino acid sequences are identified which bind to a polypeptide essential for bacterial growth or survival, these sequences can be screened for antagonist activity using, for example, the proliferation assay described above or used for screening for antagonists of said binding.

Another assay which can be performed to identify inhibitors and antagonists involves the use of combinatorial chemistry to produce random peptides which then can be screened for both binding affinity and antagonist effects. One such assay has recently been performed using random peptides expressed on the surface of a bacteriophage (Wu (1996), Nature Biotechnology 14, 429-431).

In a preferred embodiment of the method of the present invention said method further comprises identifying an antagonist or inhibitor optionally from said sample of candidate antagonists or inhibitors.

If a sample contains a candidate antagonist or inhibitor, or a plurality of candidate antagonists or inhibitors, as identified in the method of the invention, then it is either possible to isolate the candidate antagonists or inhibitors from the original sample identified as containing the compound capable of suppressing or inhibiting bacterial growth or survival, or one can further subdivide the original sample, for example, if it consists of a plurality of different candidate antagonists or inhibitors, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. As regards the identification of candidate antagonists or inhibitors by any of the above-referenced embodiments of the invention, a variety of formats or tools is available to the person skilled in the art. Thus, several methods are known to the person skilled in the art for producing and screening large libraries to identify compounds having specific affinity for a target. These methods include the phage-display method in which randomized peptides are displayed from phage and screened by affinity chromatography to an immobilized receptor; see, e.g., WO 91/17271, WO 92/01047, US-A-5,223,409. In another approach, combinatorial libraries of polymers immobilized on a chip are synthesized using photolithography; see, e.g., US-A-5,143,854, WO 90/15070 and WO 92/10092. The immobilized polymers are contacted with a labeled receptor and scanned for label to identify polymers binding to the receptor. The synthesis and screening of peptide libraries on continuous cellulose membrane supports that can be used for identifying binding ligands of the polypeptide of the invention and thus possible inhibitors and antagonists is described, for example, in Kramer, Methods Mol. Biol. 87 (1998), 25-39. This method can also be used, for example, for determining the binding sites and the recognition motifs in the polypeptide as described above. In like manner, the substrate specificity of the DnaK chaperon was determined and the contact sites between human interleukin-6 and its receptor; see Rüdiger, EMBO J. 16 (1997), 1501-1507 and Weiergraber, FEBS Lett. 379 (1996), 122-126, respectively. Furthermore, the above-mentioned methods can be used for the construction of binding supertopes derived from the polypeptide of the invention. A similar approach was successfully described for peptide antigens of the anti-p24 (HIV-1) monoclonal antibody; see Kramer, Cell 91 (1997), 799-809. A general route to fingerprint analyses of peptide-antibody interactions using the clustered amino acid peptide library was described in Kramer, Mol. Immunol. 32 (1995), 459-465. In addition, antagonists or inhibitors of a polypeptide described above can be derived and identified from monoclonal antibodies that specifically react with said polypeptide in accordance with the methods as described in Doring, Mol. Immunol. 31 (1994), 1059-1067.
More recently, WO 98/25146 described further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with the proteins according to the invention or nucleic acid molecules encoding such molecules are, for example, the <u>in vitro</u> screening with the phage display system as well as filter binding assays or "real time" measuring of interaction using, for example, the BIAcore apparatus (Pharmacia).
All these methods can be used in accordance with the present invention to identify antagonists and inhibitors of the polypeptide of the invention.

Additionally, the present invention relates in a preferred embodiment to a method comprising improving inhibitors or antagonists identified by peptidomimetics or by applying phage display or combinatorial library technique step(s). Peptidomimentics, phage display and combinatorial library techniques are well-known in the art and can be applied by the person skilled in the art without further ado to the improvement of the antagonist or inhibitor that is identified by the basic method referred to herein above.

Methods for the generation and use of peptidomimetic combinatorial libraries are described in the prior art, for example in Ostresh, Methods In Enzymology 267 (1996), 220-236; Dosner, Bioorg. Med. Chem. 4 (1996), 709-715; Beeley, Trends Biotechn. 12 (1994), 213-216; al-Obeidi, Mol. Biotechn. 9 (1998), 205-223; Wiley, Med. Res. Rev. 13 (1993), 327-384; Bohm, J. Comput. Aided Mol. Des. 10 (1996), 265-272; and Hruby, Biopolymers 43 (1997), 219-266.

Various sources for the basic structure of such an antagonist or inhibitor can be employed and comprise, for example, mimetic analogs of the polypeptide of the invention. Mimetic analogs of the polypeptide of the invention or biologically active fragments thereof can be generated by, for example, substituting the amino acids that are expected to be essential for the biological activity with, e.g., stereoisomers, i.e. D-amino acids; see e.g., Tsukida, J. Med. Chem. 40 (1997), 3534-3541. Furthermore, in case fragments are used for the design of biologically active analogs pro-mimetic components can be incorporated into a peptide to reestablish at least some of the conformational properties that may have been lost upon removal of part of the original polypeptide; see, e.g., Nachman, Regul. Pept. 57 (1995), 359-370. Furthermore, the polypeptide can be used to identify synthetic chemical peptide mimetics that bind to or can function as a ligand, substrate, binding partner or the receptor of the polypeptide as effectively as does the natural polypeptide; see, e.g., Engleman, J. Clin. Invest. 99 (1997), 2284-2292.
The structure-based design and synthesis of low-molecular-weight synthetic molecules that mimic the activity of the native biological polypeptide is further described in, e.g., Dowd, Nature Biotechnol. 16 (1998), 190-195; Kieber-Emmons, Current Opinion Biotechnol. 8 (1997), 435-441; Moore, Proc. West Pharmacol. Soc. 40 (1997), 115-119; Mathews, Proc. West Pharmacol. Soc. 40 (1997), 121-125; Mukhija, European J. Biochem. 254 (1998), 433-438.
It is also well known to the person skilled in the art, that it is possible to design, synthesize and evaluate mimetics of small organic compounds that, for example, can act as a substrate or ligand to a polypeptide as encoded by the essential gene as identified above. For example, it has been described that D-glucose mimetics of hapalosin exhibited similar efficiency as hapalosin in antagonizing multidrug resistance assistance-associated protein in cytotoxicity; see Dinh, J. Med. Chem. 41 (1998), 981-987.
The essential gene described above or the RNA encoded thereof, as has been described above, can also serve as a target for antagonists or inhibitors. Antagonists may comprise, for example, proteins that bind to the mRNA of said gene, thereby destabilizing the native conformation of the mRNA and disturbing transcription and/or translation. Furthermore, methods are described in the literature for identifying nucleic acid molecules such as an RNA fragment that mimics the structure of a defined or undefined target RNA molecule to which a compound binds inside of a cell resulting in retardation of cell growth or cell death; see, e.g., WO 98/18947 and references cited therein. These nucleic acid molecules can be used for identifying unknown compounds of pharmaceutical and/or agricultural interest, and for identifying unknown RNA targets for use in treating a disease. These methods and compositions can be used in screening for novel antibiotics, bacteriostatics, or modifications thereof or for identifying compounds useful to alter expression levels of proteins encoded by a nucleic acid molecule. Alternatively, for example, the conformational structure of the RNA fragment which mimics the binding site can be employed in rational drug design to modify known antibiotics to make them bind more avidly to the target. One such methodology is nuclear magnetic resonance (NMR), which is useful to identify drug and RNA conformational structures. Still other methods are, for example, the drug design methods as described in WO 95/35367, US-A-5,322,933, where the crystal structure of the RNA fragment can be deduced and computer programs are utilized to design novel binding compounds which can act as antibiotics.
The candidate antagonists and inhibitors which can be tested and identified according to a method of the invention may be taken from expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Furthermore, genes encoding a putative regulator of an essential bacterial protein and/or which exert their effects up- or downstream said protein may be identified using, for example, insertion mutagenesis using, for example, gene targeting vectors known in the art (see, e.g., Hayashi, Science 258 (1992), 1350-1353; Fritze and Walden, Gene activation by T-DNA tagging. In *Methods in Molecular* biology 44 (Gartland, K.M.A. and Davey, M.R., eds). Totowa: Human Press (1995), 281-294) or transposon tagging (Chandlee, Physiologia Plantarum 78 (1990), 105-115). Said compounds can also be functional derivatives or analogues of known inhibitors or antagonists. Such useful compounds can be for example transacting factors which bind an above-described polypeptide. Identification of transacting factors can be carried out using standard methods in the art (see, e.g., Sambrook, supra, and Ausubel, supra). To determine whether a protein binds to the protein or regulatory sequence of the invention, standard native gel-shift analyses can be carried out. In order to identify a transacting factor which binds to the protein or regulatory sequence of the invention, the protein or regulatory sequence of the invention can be used as an affinity reagent in standard protein purification methods, or as a probe for screening an expression library. The identification of nucleic acid molecules which encode proteins which interact with the polypeptide described above can also be achieved, for example, as described in Scofield (Science 274 (1996), 2063-2065) by use of the so-called yeast "two-hybrid system"; see also the appended example. In this system, e.g., the protein encoded by the nucleic acid molecules identified in this invention or a smaller part thereof is linked to the DNA-binding domain of the GAL4 transcription factor. A yeast strain expressing this fusion gene and comprising a lacZ reporter gene driven by an appropriate promoter, which is recognized by the GAL4 or LexA transcription factor, is transformed with a library of cDNAs which will express plant genes or fragments thereof fused to an activation domain. Thus, if a peptide encoded by one of the cDNAs is able to interact with the fusion peptide comprising a peptide of a protein of the invention, the complex is able to direct expression of the reporter gene. In this way the nucleic acid molecules and the encoded peptide can be used to identify peptides and proteins interacting with the polypeptide described above. It is apparent to the person skilled in the art that this and similar systems may then further be exploited for the identification of inhibitors or antagonists of the polypeptide.
Once the transacting factor is identified, modulation of its binding to or regulation of expression of the polypeptide described above can be pursued, beginning with, for example, screening for inhibitors against the binding of the transacting factor to the protein specified in accordance with the present invention. Inhibition of bacterial growth could then be achieved by applying the transacting factor (or its inhibitor). In addition, if the active form of the transacting factor is a dimer, dominant-negative mutants of the transacting factor could be made in order to inhibit its activity. Thus, the present invention also relates to the use of the polypeptide as defined above for the identification of antagonists or inhibitors of a polypeptide essential for bacterial growth or survival.

In another embodiment, the present invention relates to a method for designing an improved antagonist or inhibitor for the treatment of a bacterial infection or disorder or disease related to a bacterial infection comprising the steps of

(a) identification of the binding site of an antagonist or inhibitor to the polypeptide ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or identified according to the method of the present invention, by site-directed mutagenesis and chimeric polypeptide studies;

(b) molecular modeling of both the binding site of said antagonist or inhibitor and the structure of said polypeptide; and

(c) modification of said antagonist or inhibitor to improve its binding specificity or affinity for the polypeptide.

Biological assays as described above or other assays such as assays based on crystallography or NMR may be employed to assess the specificity or potency of the antagonist or inhibitor wherein the decrease of one or more activities of the polypeptide may be used to monitor said specificity or potency. All techniques employed in the various steps of the method of the invention are conventional or can be derived by the person skilled in the art from conventional techniques without further ado.

For example, identification of the binding site of said antagonist or inhibitor by site-directed mutagenesis and chimerical protein studies can be achieved by modifications in the (poly)peptide primary sequence that affect the antagonist's or inhibitor's affinity; this usually allows to precisely map the binding pocket for the drug. Identification of binding sites may be assisted by computer programs. Thus, appropriate computer programs can be used for the identification of interactive sites of a putative antagonist or inhibitor and the polypeptide of the invention by computer assisted searches for complementary structural motifs (Fassina, Immunomethods 5 (1994), 114-120).

As regards step (b), the following protocols may be envisaged: Once the effector site for antagonists or inhibitors has been mapped, the precise residues interacting with different parts of the antagonists or inhibitors can be identified by combination of the information obtained from mutagenesis studies (step (a)) and computer simulations of the structure of the binding site provided that the precise three-dimensional structure of the antagonists or inhibitors is known (if not, it can be predicted by computational simulation). If said antagonist or inhibitor is itself a peptide, it can be also mutated to determine which residues interact with others in the above-mentioned polypeptide essential for bacterial growth and survival.

Finally, in step (c) the antagonist or inhibitor can be modified to improve its binding affinity or its potency and specificity. If, for instance, there are electrostatic interactions between a particular residue of an polypeptide as defined above and some region of an antagonist or inhibitor molecule, the overall charge in that region can be modified to increase that particular interaction. Furthermore, the three-dimensional and/or crystallographic structure of inhibitors or antagonists of the polypeptide of the invention can be used for the design of peptidomimetic inhibitors or antagonists, e.g. in combination with said polypeptide (Rose, Biochemistry 35 (1996), 12933-12944; Rutenber, Bioorg. Med. Chem. 4 (1996), 1545-1558).

Potential antagonists/inhibitors include antisense molecules. Antisense technology can be used to control gene expression through antisense DNA or through triple-helix formation. Antisense techniques are discussed, for example, in Okano, J. Neurochem. 56 (1991), 560; Oligodeoxynucleotides as Antisense Inhibitors of Gene Expression, CRC Press, Boca Raton, FL (1988). Triple helix formation is discussed in, for instance, Lee, Nucl. Acids Res. 6 (1979), 3073; Cooney, Science 241 (1988), 456; and Dervan, Science 251 (1991), 1360. The methods are based on binding of a polynucleotide to a complementary DNA or RNA.

For example, the 5' coding portion of a polynucleotide that encodes the mature polypeptide as described above may be used to design an antisense RNA oligonucleotide of from about 10 to 40 base pairs in length. A DNA oligonucleotide is designed to be complementary to a region of the gene involved in transcription thereby preventing transcription and the production of the protein. The antisense RNA oligonucleotide hybridizes to the mRNA and blocks translation of the mRNA molecule into receptor polypeptide. As indicated, antagonist or inhibitor e.g. polyclonal and monoclonal antibody according to the teachings of the present invention can be raised according to the methods disclosed in Tartaglia, J. Biol. Chem. 267 (1992), 4304-4307; Tartaglia, Cell 73 (1993), 213-216, and PCT Application WO 94/09137.

Antibodies may be prepared by any of a variety of methods using immunogens of the polypeptide described above. As indicated, such immunogens include the full length polypeptide (which may or may not include the leader sequence) and fragments such as the ligand binding domain, the extracellular domain and the intracellular domain. These antibodies can be monoclonal antibodies, polyclonal antibodies or synthetic antibodies as well as fragments of antibodies, such as $Fab^+$, Fv, $F(ab')_2$, disulphide-bridged Fv or scFv fragments, etc. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals. Furthermore, antibodies or fragments thereof to the aforementioned peptides can be obtained by using methods which are described, e.g., in Harlow and Lane "Antibodies, A Laboratory Manual", CSH Press, Cold Spring Harbor, 1988.

The antagonists or inhibitors isolated by the above methods also serve as lead compounds for the development of analog compounds. The analogs should have a stabilized electronic configuration and molecular conformation that allows key functional groups to be presented to the receptor in substantially the same way as the lead compound.

In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art. Furthermore, peptidomimetics and/or computer aided design of appropriate derivatives and analogues can be used, for example, according to the methods described above.

The inhibitor or antagonist identified by the above-described method may prove useful as a pesticide, and/or antibiotic. The inhibitors and antagonists of the present invention preferably have a specificity at least substantially identical to the binding specificity of the natural ligand or binding partner of the polypeptide described above. An antagonist or inhibitor can have a binding affinity to said polypeptide of at least $10^5M^{-1}$, preferably higher than $10^7M^{-1}$ and advantageously up to $10^{10}M^{-1}$. In a preferred embodiment, an inhibitor, e.g. suppressive antibody, has an affinity of at least about $10^{-7}$ M, preferably at least about $10^{-9}$ M and most preferably at least about $10^{-11}$ M; and the antagonist has an affinity of less than about $10^{-7}$ M, preferably less than about $10^{-9}$ M and most preferably in order of $10^{-11}$M. In the case of nucleic acid molecules it is preferred that they have a binding affinity to those encoding the amino acid sequences depicted in any one of SEQ ID NOS: 16 to 37 of at most 2-, 5- or 10-fold less than an exact complement of 20 consecutive nucleotides of the above described nucleic acid molecules.

In another embodiment, the present invention relates to a method for producing a therapeutic agent comprising synthesizing the above-described antagonist or inhibitor.

Preferably, the compound identified according to the above described method or its analog or derivative is further formulated in a therapeutically active form or in a form suitable for the application against bacterial infections or diseases related to such an infection. For example, it can be combined with a pharmaceutically acceptable carrier known in the art. Thus, the present invention also relates to a method of producing a (therapeutically effective) composition comprising the steps of one of the above described methods of the invention and combining the compound obtained or identified in the method of the invention or an analog or derivative thereof with a pharmaceutically acceptable carrier.

Also, the present invention relates to a composition comprising the antagonist or inhibitor mentioned above. As is evident from the above, the present invention generally relates to compositions comprising at least one of the aforementioned antagonists or inhibitors, which may be nucleic acid molecules, proteins or antibodies,. Advantageously, said composition is for use as a medicament, a diagnostic means, or a kit.

The term "composition", as used in accordance with the present invention, comprises at least one small molecule or molecule as identified herein above, such as a protein, an antigenic fragment of said protein, a fusion protein, a nucleic acid molecule and/or an antibody as described above and, optionally, further molecules, either alone or in combination, like e.g. molecules which are capable of optimizing antigen processing, cytokines, immunoglobulins, lymphokines or CpG-containing DNA stretches or, optionally, adjuvants. The composition may be in solid, liquid or gaseous form and may be, inter alia, in form of (a) powder(s), (a) tablet(s), (a) solution(s) or (an) aerosol(s). In a preferred embodiment, said composition comprises at least two, preferably three, more preferably four, most preferably five differentially synthesized proteins.

The antagonists and inhibitors of the invention appear to function against gene products which are essential in several strains or genera of bacteria. Accordingly, the above-described antagonists and inhibitors may be used to inhibit the growth of a wide spectrum of bacteria. The above described antagonists or inhibitors may be used to slow, stop, or reverse bacterial growth. Thus, the present invention also relates to a method of producing a therapeutic agent comprising the steps of the methods described hereinbefore and synthesizing the antagonist or inhibitor obtained or identified as described above or an analog or derivative thereof, preferably in an amount sufficient to provide said agent in a therapeutically effective amount to a patient.

Compounds identified by the above methods or analogs are formulated for therapeutic use as pharmaceutical compositions. The compositions can also include, depending on the formulation desired, pharmaceutically acceptable, usually sterile, non-toxic carriers or diluents, which are defined as vehicles commonly used to formulate pharmaceutical compositions for animal or human administration. The diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like. A therapeutically effective dose refers to that amount of protein or its antibodies, antagonists, or inhibitors which ameliorate the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg per dose; however, doses below or above this exemplary range are envisioned, especially considering the aforementioned factors. Administration of the suitable compositions may be effected by different ways, e.g., by intravenous, intraperitoneal, subcutaneous, intramuscular, topical or intradermal administration. If the regimen is a continuous infusion, it should also be in the range of 1 µg to 10 mg units per kilogram of body weight per minute, respectively. Progress can be monitored by periodic assessment. The compositions of the invention may be administered locally or systemically. Administration will generally be parenterally, e.g., intravenously. The compositions of the invention may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as interleukins, interferons and/or CpG-containing DNA stretches, depending on the intended use of the pharmaceutical composition.

In another embodiment, the present invention relates to a kit comprising at least one of the aforementioned antagonists or inhibitors of the invention. The kit of the invention as well as the composition may in a preferred embodiment contain further ingredients such as selection markers, antibiotics, cytokines and components for simplifying or supporting the treatment of bacterial infections or disorders or diseases related to bacterial infections. The kit of the invention may advantageously be used for carrying out the method of the invention and could be, inter alia, employed in a variety of applications referred to herein, e.g., in the diagnostic field or as research tool. The parts of the kit of the invention can be packaged individually in vials or in combination in containers or multicontainer units. Manufacture of the kit follows preferably standard procedures which are known to the person skilled in the art. The kit or its ingredients according to the invention can be used in antibacterial therapies, for example, for any of the above described methods for detecting further inhibitors and antagonists essential for bacterial growth and survival. The kit of the invention and its ingredients are expected to be very useful for the healing and protection of animals and humans suffering from a bacterial infection.

The present invention also relates to a method for treating or preventing bacterial infections or diseases or disorders related to bacterial infections comprising the step of administering to a subject in need thereof an antagonist or inhibitor identified herein above, optionally comprised in a pharmaceutical composition of the invention.

In another embodiment the present invention relates to the use of a polypeptide encoded by the gene as identified above or a fragment, derivative or ortholog thereof or of any of said genes for the identification of an antagonist or inhibitor of said polypeptide fragment, derivate or ortholog or said gene.

In a further embodiment the present invention relates to the use of said polypeptide, the therapeutic agent produced according to the invention, the antagonist or inhibitor obtained or identified by the method or use according to the invention for the preparation of a pharmaceutical composition for the treatment of (a) bacterial infection(s), disorder(s) and/or disease(s) related to bacterial infections.

In another embodiment the present invention relates to a method for treating or preventing bacterial infections or diseases or disorders related to bacterial infections comprising the step of administering to a subject in need thereof an antagonist or inhibitor identified herein above, optionally comprised in the pharmaceutical composition according to the present invention.

In a further embodiment the present invention relates to the use of the above-described polypeptide, a fragment, derivative or ortholog thereof or of any of said genes for screening for polypeptides interacting with said polypeptide using protein-protein interaction technologies, and/or for validating such interaction as being essential for bacterial survival and/or for screening for antagonists or inhibitors of such interaction.

In a further embodiment the present invention relates to the use of the above-described polypeptide, a fragment, derivative or ortholog thereof or of any of said genes for screening of polypeptide for polypeptide binding to said polypeptide, and/or for validating the peptides binding to said polypeptide as preventing growth of bacteria or being lethal to bacteria upon expression of said polypeptides in said bacteria, and/or for screening for small molecules competitively displacing said peptides.

In another embodiment the present invention relates to the use of a conditional mutant of a gene as described above or a fragment, derivative or ortholog thereof or of surrogate ligands against said gene expressed in bacteria to induce a lethal phenotype in bacteria and/or for the analysis of said bacteria for surrogate markers by comparison of RNA or protein profiles in said bacteria with RNA or protein profiles in wild type bacteria, and/or the use of said surrogate markers for the identification of antagonists of the essential function of said gene.

In another embodiment the present invention relates to a method for identifying or isolating a surrogate marker comprising the steps as described in the above-recited method of the present invention.

In a further embodiment the present invention relates to a method for identifying or isolating a surrogate marker comprising the steps of

(a) inducing a lethal phenotype in bacteria representing a conditional mutant of a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD; and

(b) analysing said bacteria comparing the RNA or protein profile of said bacteria with wild type bacteria.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.ti gr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

The present invention is further illustrated by reference to the following non-limiting examples.

Unless stated otherwise in the examples, all recombinant DNA techniques are performed according to protocols as described in Sambrook et al. (1989), Molecular Cloning : A Laboratory Manual. Cold Spring Harbor Laboratory Press, NY or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, jointly published by BIOS Scientific Publications Ltd. (UK) and Blackwell Scientific Publications (UK).

**Brief description of the figures**

Figure 1: Sequences of the essential bacterial genes identified according to the method described in the examples

Figure 2: PCR strategy and the position of primers used

Figure 3: Sequence comparison table of essential E.coli genes with proposed orthologs from various bacteria. Unfinished genomes are indicated by asterisk. Complete genomes were analysed using BlastP2. Unfinished genomes were analysed with TBlastN. Orthologous sequences can be accessed at the respective WWW links as indicated in the footnotes.

**Example 1**

Automated BLASTP-based genome comparisons to identify *E. coli* FUN genes resulted in the following list of 65 candidate genes which are conserved between *E. coli, B. subtilis, H. influenzae, H. pylori, M. tuberculosis, Ch. trachomatis, B. burgdorferi, T. pallidum, S. pneumoniae, S. aureus, E. faecalis, P. aeruginosa, B. pertussis* and which were further analysed:

| FUN Genes | Gene Bank Accession Number | FUN Genes | Gene Bank Accession Number | FUN Genes | Gene Bank Accession Number |
|---|---|---|---|---|---|
| ygbB | g1789103 | yggS | g1789321 | yaeE | g1786397 |
| yhaD | g1789512 | yggV | g1789324 | yicC | g1790075 |
| yhbU | g1789548 | yggW | g1789325 | yebK | g1788159 |
| yhiN | g2367234 | yjhG | g2367371 | yhbC | g1789561 |
| yieG | g1790150 | yjiR | g1790797 | ygbP | g1789104 |
| yihZ | g1790320 | yohI | g1788462 | ybaX | g1786648 |
| yjgF | g1790691 | yqhThom | g1788728 | yqcD | g1789158 |
| yacE | g1786292 | yfiH | g1788945 | ybeY | g1786880 |
| yaeC | g1786396 | yhaR | g1789501 | gcpE | g1788863 |
| yagF | g1786464 | yhdG | g1789660 | kdtB | g1790065 |
| ybeB | g1786856 | yccG | g1787197 | pfs | g1786354 |
| ycfH | g147382 | ychB | g1787459 | sms | g1790850 |
| ydcP | g1787705 | yejD | g1788510 | ycaJ | g1787119 |
| ydiB | g1787983 | yidD | g140861 | yhhF | g1789875 |
| yebI | g1788166 | yrfI | g1789804 | yleA | g1786882 |
| yeeC | g1788320 | yggJ | g1789315 | b1808 | g1788110 |
| yegQ | g1788397 | yjeE | g1790610 | yeaA | g1788077 |
| yfcB | g1788670 | yiaO | g1790004 | b1675 | g1787964 |
| yfgB | g1788865 | yrdC | g2367210 | yhbU/yegQ[a] | g1789548 / g1788397 |
| yfhC | g1788911 | b1983 | g1788294 | yjgF/yhaR[a] | g1790691 / g1789501 |
| ygcA | g1789148 | smpB | g1788973 | yohI/yhdG[a] | g1788462 / g1789660 |
| ygfA | g1789278 | ydiD | g1787993 | | |

[a]: double mutants were created when the respective genes were paralogues in *E.coli*

**Creating in-frame deletions of *E. coli* genes**

The subsequent description of the construction of deletion mutants was carried out essentially equal for all 65 candidate genes. Particular details will exemplarily be described for one gene which gave rise to be **essential (yfhC)** and one which was **non-essential (yggV)**.

**1) Principle of the PCR-procedure and primer-design for *in frame* deletions:**

Unless an overlapping ORF exists, primers dgenX2 and dgenX3 are designed to delete the entire ORF from ATG to STOP, e.g.: ATGgttataaatttggagtgtgaaggttattgcgtgTAA (SEQ ID NO: 1) (see figure). The 5'-ends of primers dgenX1 and dgenX4 contain random nucleotides followed preferably by a BamHI site (dgenX1) or a SalI site (dgenX4) for cloning into plasmid pKO3 (Link et al (1997), J Bac 179: 6228-6237). In most mutants, primers dgenX2 and dgenX3 contain a 33 bp tag sequence called "Church-tag".

Church-tag forward direction: 5'-gttataaatttggagtgtgaaggttattgcgtg-3' (SEQ ID NO: 2)

Church-tag reverse direction: 5'-cacgcaataaccttcacactccaaatttataac-3' (SEQ ID NO: 3)

This tag is used for a subsequent PCR in which the 5'- and 3'- flanking DNA-fragments of the deletion construct are assembled.

In the few constructs lacking the "Church-tag", the primers dgenX2 and dgenX3 carry at their 5'-ends 5 random nucleotides followed by a restriction site (preferably EcoRI) which by its positioning creates the *in frame* deletion.

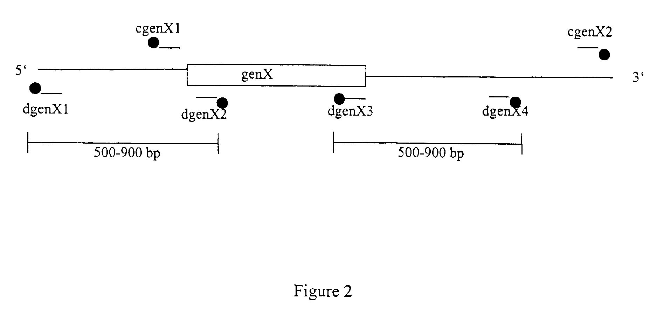

Oligos cgenX1 and cgenX2 are used for the verification of the chromosomal situation (wild type or deletion) after the replacement procedure (Fig. 2).

Primers for the respective candidate genes were designed as follows:

dyfhC1: 5'-GATCGGATCCAAATTCCAGTTAGCCATGATGCGGTC-3' (SEQ ID NO: 4)

dyfhC2: 5'-CACGCAATAACCTTCACACTCCAAATTTATAACCATTATA CACGGACGCTATGC-3' (SEQ ID NO: 5)

dyfhC3: 5'-GTTATAAATTTGGAGTGTGAAGGTTATTGCGTGACGGATTAATT TTGTTTCTCTT-3' (SEQ ID NO: 6)

dyfhC4: 5'-GATCGTCGACGCGCTCGATATCACCGATGAACAACCG-3' (SEQ ID NO: 7)

cyfhC1: 5'-CAATCCGCTGCTTTATTTCTGTCAG-3' (SEQ ID NO: 8)

cyfhC2: 5'-TTATAACGAAATCAACGGGAAACCT-3' (SEQ ID NO: 9)

dyggV1: 5'-GATCGGATCCCTCTAAAAAATAAGGAATTAAAGG-3' (SEQ ID NO: 10)

dyggV2: 5'-CACGCAATAACCTTCACACTCCAAATTTATAACCATAGGATAC CTAATTAATTAAC-3' (SEQ ID NO: 11)

dyggV3: 5'-GTTATAAATTTGGAGTGTGAAGGTTATTGCGTGAAGAGCGCC ATTTCCCACCGT-3' (SEQ ID NO: 12)

dyggV4: 5'-GATCGTCGACTCATATTGCTGATAACCCGCTGCGGT-3' (SEQ ID NO: 13)

cyggV1: 5'-GTTGACGGCCAGGCCAACAGTCAT-3' (SEQ ID NO: 14)

cyggV2: 5'-ATAACCCTGGCAATCGCCTCG-3' (SEQ ID NO: 15)

**Example 2**

**Construction of the DNA-fragments comprising the deletion**

**The 5'- and the 3'-flanking DNA fragments are PCR amplified in a total volume of 50 μl as follows:**

| Chromosomal DNA from *E. coli* strain MG1655 (100 ng/μl): | final conc.: 1 ng/μl |
|---|---|
| 10*Pwo-buffer | final conc.: 1x |
| dgenX1/3 (10 μM) | final conc.: 500 nM |
| dgenX2 (4) (10 μM) | final conc.: 500 nM |
| Pwo-Polymerase | final conc.: 5 U/100 μl |
| dNTPs (25 mM) | final conc.: 250 μM |
| H20 | to adjust volume to 50 μl |

PCR conditions:

4' 94 °C
30 cycles: 30" 94 °C, 30" 44 °C, 1' 72 °C
5' 72 °C

The PCR products are then purified with the High Pure PCR Purification Kit (Boehringer) to remove salts and enzyme (elute in 50 μl $H_2O$). Alternatively, if PCR products contain prominent impurities, the respective fragment must be purified by agarose gel extraction (Gene Clean, Dianova) before the fragment assembly.

**Assembly PCR**

Equal amounts of 5'- and 3'-fragment are applied as template DNA. In general a volume applied for gel electrophoresis giving an intense band is o.k. The total reaction volume is 100 μl. For the assembly the "outer" primers dgenX1 and dgenX4 were used.

| | |
|---|---|
| 5'-Fragment | approx. 10 ng |
| 3'-Fragment | approx. 10 ng |
| 10*Pwo-buffer | final conc.: 1x |

(continued)

| | |
|---|---|
| dgenX1 (10 μM) | final conc.: 500 nM (50 pmol/100 μl) |
| dgenX4 (10 μM) | final conc.: 500 nM (50 pmol/100 μl) |
| Pwo-Pol (Boehringer) | final conc.: 5 U/100 μl |
| dNTPs (25mM) | final conc.: 250 μM |
| $H_2O$ | add to 100 μl |

**PCR conditions:**

4' 94 °C
10 cycles: 30" 94 °C, 30" 44 °C, 1' 72 °C
25 cycles: 30" 94 °C, 30" 44 °C, 3' 72 °C
5' 72 °C

The success of the PCR is checked by agarose gel electrophoresis. The assembled PCR product is purified with the High Pure PCR Purification Kit and the complete eluate of 50 μl is over-night digested with BamHI and SalI in a volume of 60 μl. After gel electrophoresis the digested product is purified with Gene Clean (Dianova) to remove small oligonucleotides quantitatively (elution volume: 25 μl).

**Cloning into vector pKO3:**

Next, the fragment is ligated into the vector pKO3 (cut with BamHI and SalI) in a 10-20 μl reaction (T4-DNA ligase) for 2 hours at room temperature.

**Transformation into DH5:**

One half of the ligation mix is transformed into chemically competent *E. coli* DH5α and clones are purified once (usually 8 clones are sufficient).

**Verification of deletion constructs:**

1) 8 clones are characterized by colony-PCR with vector pKO3-specific primers (pKO3-B1 and pKO3-S1).
2) Clones with the correct size of insert are double-checked by colony-PCR with gene specific primers (dgenX1 and dgenX4).

**Reaction mixture for 25 μl reaction volume:**

| | |
|---|---|
| template (colony) | 1 μl of 1 colony resuspended in 20 μl $H_2O$ |
| 10*Taq-buffer | final conc.: 1x |
| 5*Q-solution | final conc.: 1x |
| pKO3-B1/dgenX1 (100 μM) | final conc.: 1 μM (50 pmol/100 μl) |
| pKO3-S1/dgenX4 (100 μM) | final conc.: 1 μM (50 pmol/100 μl) |
| Taq-Pol (QIAgen) | final conc.: 2 U/25 μl |
| dNTPs (25 mM) | final conc.: 250 μM |
| $H_2O$ | 15.35 μl |

**PCR conditions:**

4' 94 °C
25 cycles: 30" 94 °C, 30" 50 °C, 2' 65 °C
5' 65 °C

3) Plasmid-DNA from 4 ml over-night culture is prepared using a QIAgen Miniprep Kit and a double restriction analysis with BamHI/SalI and EcoRI/HindIII is performed to verify the clones.

**Protocol referring to the construction of assembly products by a restriction site:**

The 5'- and the 3'-fragments are PCR amplified as described above. The PCR products are purified with the High Pure PCR Purification Kit (Boehringer) to remove salts and enzyme and 5 to 10 µl are digested over night using the restriction site creating the deletion (primers 2 and 3; mostly EcoRI) in a total volume of 30 µl. The restriction products are again purified with the High Pure PCR Purification Kit to remove nucleotides, salts and enzyme. (Alternatively: Following preparative agarose gel electrophoresis the cut fragments are isolated using Gene Clean (Dianova) and eluted in a volume of 25 µl. The cut fragments (3-6 µl each) are ligated in a volume of 10-15 µl using T4-DNA ligase for 2 hours at room temperature. 5 µl of this ligation mix is directly used as a template for a second PCR. In this PCR, the assembled fragments are amplified using primers dgenX1 and dgenX4. The reaction is set up as described above with two exceptions: 1) The total reaction volume is 100 µl and 2) the extension step at 72 °C lasts 3'.

**Example 3**

**The chromosomal exchange strategy**

(Link et al (1997), J Bac 179: 6228-6237)

**Cointegration:**

Cointegration = integration of a plasmid into the chromosome by a recombination event
The pKO3 derivative is transformed into MG1655 or any recA+ strain

**Day 1**

The strain is grown at 30 °C in LB containing 20 µg/ml chloramphenicol (LB-Cam20) to an $OD_{600}$ of ~1.0. Afterwards, perform 10-fold serial dilutions in the same medium (down to $10^{-7}$). For the following plating use prewarmed LB-Cam20 agar plates. Plate 100 µl of dilutions $10^{-4}$ and $10^{-5}$ for incubation at 44 °C and 100 µl of dilutions $10^{-6}$ and $10^{-7}$ for incubation at 30 °C.

**Day 2**

Following incubation at the respective temperature, determine the factor c.f.u.44 °C/c.f.u.30 °C (c.f.u. = colony forming units). This factor for pKO3 without insert is in the range $1*10^{-4}$ to $5*10^{-4}$ and should be significantly larger in the case of successful cointegration. Purify 8 randomly chosen clones from the 44 °C plate twice on LB-Cam20 agar plates at 44 °C (during Day 2 and over night to Day 3). Optionally, confirm the clones for their identity as cointegrates by colony-PCR.

**Resolution and counter-selection:**

Resolution = resolution of the cointegrate resulting in a self replicative plasmid by a second recombination event
Counter-selection = selection <u>against</u> the presence of plasmid in the cell

**Day 3**

Pool single colonies from each of the 8 cointegrates in 100 µl LB and use this suspension as an inoculum for 10 ml LB+5 %sucrose. After growth at 30 °C (8 to 10 hours during a day is sufficient) 10-fold serial dilutions are performed and 100 µl of dilutions $10^{-4}$, $10^{-5}$, and $10^{-6}$ are plated onto LB agar+5 % sucrose and grown over night at 30 °C.

**Day 4**

50 single colonies are replica streaked on LB+Cam20 and LB+5 % sucrose to test for the loss of plasmid.

**Example 4**

**Testing for essentiality of FUN genes of *E. coli* and interpretation of the results**

**Day 5**

The clones sensitive to chloramphenicol are then tested for their genotype (wild type versus in-frame deletion) by colony-PCR using primers cgenX1 and cgenX2 (10-48 clones).

In the case of the gene **yfhC** out of 48 clones tested **only wild type** situation on the chromosome could be detected.

In the case of the gene **yggV** out of 48 clones **16 (= 33 %)** revealed a PCR product with a size indicative for the **deletion** situation on the chromosome.

Are 48 clones revealing no mutant enough to claim a gene as essential? This question can be answered by asking for the number of clones that have to be tested to get a confidence of e.g. 99 % that really no mutants are present in an infinite number of clones. Provided a hypothesis Ho means that only the wild type genotype is viable and hypothesis $H_1$ means that a fraction (1-x) of mutants is allowed to occur together with the wild type (x) among a population of clones (x + (1-x)), then the probability to make the wrong decision (decision for $H_0$ whereas $H_1$ is true) can be calculated as

$$(1) \qquad x^n/(1+x^n)$$

where x is the fraction of wild type clones and n is the number of clones tested. The confidence niveau a to make the wrong decision (error probability) is given by

$$(2) \qquad \alpha > x^n/(1+x^n)$$

thereby resulting in

$$(3) \qquad n > \ln(\alpha / (1-\alpha)) / \ln(x)$$

for the number of clones that have to be tested to prove or disprove hypothesis $H_0$.

If the average probability for obtaining wild type clones (x) in a replacement experiment is 70 % (experimentally determined for 43 non-essential genes out of 65 candidate genes), then, after testing of 26 clones which reveal a wild type genotype an uncertainty of 0.01 % error probability (α) remains that the claiming of a gene as essential could be wrong. Even if the rate of obtaining wild types (x) is set to 85 % (a value which occurs with a frequency of 10 % for replacement experiments with non-essential genes), then, by testing 32 clones (which was performed in every experiment giving rise to an essential gene) an error probability of only 0.6 % remains to chose the wrong hypothesis.

**Examle 5**

**List of essential FUN genes obtained**

By the described method the following 22 genes were obtained which gave no deletion genotype and are therefore claimed to be essential:

| *E. coli* | |
|---|---|
| gene name | GenBank# |
| ygbB | g1789103 |
| yfhC | g1788911 |
| yacE | g1786292 |
| ychB | g1787459 |
| yejD | g1788510 |
| yrfl | g1789804 |
| yggJ | g1789315 |
| yjeE | g1790610 |
| yiaO | g1790004 |
| yrdC | g2367210 |
| yhbC | g1789561 |
| ygbP | g1789104 |
| ybeY | g1786880 |
| gcpE | g1788863 |
| kdtB | g1790065 |
| pfs | g1786354 |
| ycaJ | g1787119 |
| b1808 | g1788110 |
| yeaA | g1788077 |
| yagF | g1786464 |
| b1983 | g1788294 |
| yidD | g140861 |

## Claims

1. A method for identifying an antagonist or inhibitor of the expression of a gene encoding a polypeptide essential for bacterial growth or survival wherein said gene is selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or a fragment, derivative or ortholog thereof, said method comprising the steps of

   (a) testing a candidate antagonist or inhibitor or a sample comprising a plurality of said candidate antagonists or inhibitors for the inhibition or reduction of transcription of said gene or a fragment or derivative thereof; or
   (b) testing a candidate antagonist or inhibitor or a sample comprising a plurality of said candidate antagonists or inhibitors for the inhibition or reduction of translation of mRNA transcribed from said gene or a fragment or derivative thereof; and
   (c) identifying an antagonist or inhibitor or a sample comprising a plurality of said candidate antagonists or inhibitors that tests positive in step (a) and/or (b).

2. A method for testing a candidate antagonist or inhibitor of a polypeptide or a mRNA essential for bacterial growth or survival encoded by a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or a fragment, derivative or ortholog thereof comprising the steps of

   (a) contacting a bacterial cell with a candidate antagonist or inhibitor or a sample comprising a plurality of said candidate antagonists or inhibitors; and
   (b) testing whether said contacting leads to cell growth inhibition and/or cell death.

3. A method for testing a candidate antagonist or inhibitor of the function of a gene essential for bacterial growth or survival wherein said gene is selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or a fragment, derivative or ortholog thereof, comprising the steps of

   (a) contacting a bacterial cell comprising said gene with a candidate antagonist or inhibitor or a sample comprising a plurality of said candidate antagonists or inhibitors; and
   (b) testing whether said contacting leads to cell growth inhibition and/or cell death.

4. The method of any one of claims 1 to 3 further comprising identifying an antagonist or inhibitor, optionally from said sample of candidate antagonists or inhibitors.

5. The method of any one of claims 1 to 4 wherein said inhibitor or antagonist is further improved by peptidomimetics or by applying phage display or combinatorial library technique step(s).

6. A method for designing an improved antagonist or inhibitor for the treatment of a bacterial infection or disorder or disease related to a bacterial infection comprising the steps

   (a) identification of the binding site of an antagonist or inhibitor to the polypeptide ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or obtained by or identified by the method of any one of claims 1 to 5 by site-directed mutagenesis and chimeric polypeptide studies;
   (b) molecular modeling of both the binding site of said antagonist or inhibitor and the structure of said polypeptide; and
   (c) modification of said antagonist or inhibitor to improve its binding specificity or affinity for the polypeptide.

7. An antagonist or inhibitor of the activity of a polypeptide encoded by a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or fragment, derivative or ortholog thereof or of the expression of a gene encoding said polypeptide or said fragment, derivative or ortholog or obtained by or identified by the method of any one of claims 1 to 6.

8. A method for producing a therapeutic agent comprising synthesizing the antagonist or inhibitor identified, tested or designed according to the method of any one of claims 1 to 6 or the antagonist or inhibitor of claim 7 or an analog or derivative thereof.

**9.** A method for producing a composition comprising the steps of the method of any one of claims 1 to 6 or synthesizing the antagonist or inhibitor of claim 7 and formulating said inhibitor or antagonist in a pharmaceutically acceptable form.

**10.** A composition comprising an antagonist or inhibitor of claim 7, the therapeutic agent produced by the method of claim 8 or the antagonist or inhibitor obtained by or identified in the method of any one of claims 1 to 6 or produced according to claim 9 and optionally a pharmaceutically acceptable carrier.

**11.** The composition of claim 10 which is a pharmaceutical composition.

**12.** The composition of claim 10 which is a kit.

**13.** The composition of any one of claims 10 to 12 further comprising an antibiotic and/or cytokine.

**14.** Use of a polypeptide encoded by a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or a fragment, derivative or ortholog thereof or of any of said genes for the identification of an antagonist or inhibitor of the activity of said polypeptide or said fragment, derivative or ortholog or of the expression of a gene encoding said polypeptide or said fragment, derivative or ortholog.

**15.** Use of an antagonist or inhibitor of claim 7, the therapeutic agent produced by the method of claim 8 or the antagonist or inhibitor obtained by or identified in the method of any one of claims 1 to 6 or produced according to claim 9 or identified by the use of any of the claims for the preparation of a pharmaceutical composition for the treatment of (a) bacterial infection(s), disorder(s) and/or disease(s) related to bacterial infections.

**16.** A method for treating or preventing bacterial infections or diseases or disorders related to bacterial infections comprising the step of administering to a subject in need thereof the antagonist or inhibitor obtained by or identified in the method of any one of claims 1 to 6 or produced according to claim 9 optionally comprised in the pharmaceutical composition according to claim 11.

**17.** Use of a polypeptide encoded by a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or a fragment, derivative or ortholog thereof or any of said genes for screening for polypeptides interacting with said polypeptide using protein-protein interaction technologies, and/or for validating such interaction as being essential for bacterial survival and/or for screening for antagonists or inhibitors of such interaction.

**18.** Use of a polypeptide encoded by a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or a fragment, derivative or ortholog thereof or any of said genes for screening of polypeptide for polypeptide binding to said polypeptide, and/or for validating the peptides binding to said polypeptide as preventing growth of bacteria or being lethal to bacteria upon expression of said polypeptides in said bacteria, and/or for screening for small molecules competitively displacing said peptides.

**19.** Use of conditional mutants in a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1, or a fragment, derivative or ortholog thereof or of surrogate ligands against said gene expressed in bacteria to induce a lethal phenotype in bacteria and/or for the analysis of said bacteria for surrogate markers by comparison of RNA or protein profiles in said bacteria with RNA or protein profiles in wild type bacteria, and/or the use of said surrogate markers for the identification of antagonists of the essential function of said gene.

**20.** A method for identifying or isolating a surrogate marker comprising the steps as described in claim 19.

**21.** A method for identifying or isolating a surrogate marker comprising the steps of

(a) inducing a lethal phenotype in bacteria containing a conditional mutant of a gene selected from the group consisting of ygbB, yfhC, yacE, ychB, yejD, yrfl, yggJ, yjeE, yiaO, yrdC, yhbC, ygbP, ybeY, gcpE, kdtB, pfs, ycaJ, b1808, yeaA, yagF, b1983 and/or yidD, the sequence of said genes being shown in Fig. 1; and
(b) analysing said bacteria comparing the RNA or protein profile of said bacteria with wild type bacteria.

# Figure 1

1) ygbB
atgcgaattggacacggttttgacgtacatgcctttggcggtgaaggcccaattatcattggtggcgtacgcattccttacgaaaaggattgctg
gcgcattctgatggcgacgtggcgctccatgcgttgaccgatgcattgcttggcgcggcggcgctggggatatcggcaagctgttcccggata
ccgatccggcatttaaaggtgccgatagccgcgagctgctacgcgaagcctggcgtcgtattcaggcgaagggttatacccttggcaacgtcg
atgtcactatcatcgctcaggcaccgaagatgttgccgcacattccacaaatgcgcgtgtttattgccgaagatctcggctgccatatggatgatgt
taacgtgaaagccactactacggaaaaactgggatttaccggacgtggggaagggattgcctgtgaagcggtggcgctactcattaaggcaa
caaaatga (SEQ ID NO: 16)

2) yfhC
atgcgccgcgcttttataaccggagttttctttttgtctgaagtcgaatttagccacgaatactggatgcgtcacgcgctgacgctggcgaaacgtg
cctgggatgagcgggaagtgccggtcggcgcggtattagtgcataacaatcgggtaatcggcgaaggctggaaccgcccgattggtcgccat
gatcccaccgcacatgcagaaatcatggccctgcggcagggtggtctggtgatgcaaaattatcgtctgatcgacgccacgttgtatgtcacgct
tgaaccatgtgtaatgtgtgccggagcgatgatccacagtcgcattggtcgcgtggtctttggtgcgcgtgacgcgaaaactggcgctgcgggat
ctttaatggatgtgctgcatcatccgggtatgaatcaccgagtggaaattacggaaggaatactggcggatgagtgcgcggcgttgctcagtga
cttctttcgcatgcgccgccaggaaattaaagcgcagaaaaaagcgcaatcctcgacggattaa (SEQ ID NO: 17)

3) yacE
atgaggtatatagttgccttaacgggaggcattggcagtggcaagagtaccgttgccaatgcgtttgctgatctcggaattaacgtcattgatgcc
gatattattgcgcgtcaggtggttgaaccaggtgcacctgcgctacatgccattgctgatcactttggcgctaacatgattgctgctgatggaacatt
gcagcgccgggccttgcgcgagcggatcttcgccaacccggaagagaaaaactggcttaacgccctgctgcatccgctgattcagcaagag
acgcaacaccagatccagcaagctacttcccccctatgtactgtgggttgtgccattgctggtagaaaactcactgtataaaaaagcgaatcgag
tgcttgtggtggatgtcagcccagaaacgcaacttaagcgcaccatgcagcgcgatgatgtaactcgcgagcatgtcgaacaaatccttgctgc
tcaggcaacgcgcgaagcccgccttgccgtggcagatgacgtcattgataataacggcgcaccggatgctatcgcatcggatgttgcccgcct
gcacgcacactatttgcagcttgcgtcgcagtttgtctcacaggaaaaaccgtaa (SEQ ID NO: 18)

4) ychB
atgcggacacagtggccctctccggcaaaacttaatctgtttttatacattaccggtcagcgtgcggatggttaccacacgctgcaaacgctgtttc
agtttcttgattacggcgacaccatcagcattgagcttcgtgacgatggggatattcgtctgttaacgcccgttgaaggcgtggaacatgaagata
acctgatcgttcgcgcagcgcgattgttgatgaaaactgcggcagacagcgggcgtcttccgacgggaagcggtgcgaatatcagcattgac
aagcgtttgccgatgggcggcggtctcggcggtggttcatccaatgccgcgacggtcctggtggcattaaatcatctctggcaatgcgggctaa
gcatggatgagctggcggaaatggggctgacgctgggcgcagatgttcctgtctttgttcgggggcatgccgcgtttgccgaaggcgttggtga
aatactaacgccggtggatccgccagagaagtggtatctggtggcgcaccctggtgtaagtattccgactccggtgatttttaaagatcctgaact
cccgcgcaatacgccaaaaaggtcaatagaaacgttgctaaaatgtgaattcagcaatgattgcgaggttatcgcaagaaaacgttttcgcga
ggttgatgcggtgctttcctggctgttagaatacgccccgtcgcgcctgactgggacaggggcctgtgtctttgctgaatttgatacagagtctgaa
gcccgccaggtgctagagcaagccccggaatggctcaatggctttgtggcgaaaggcgctaatctttccccattgcacagagccatgctttaa
(SEQ ID NO: 19)

5) yejD
atgcgacttgataaatttatcgcacagcaactcggcgttagccgtgctattgccgggcgtgaaatccgcggcaatcgtgtcaccgtcgatggcga
aatcgtccgtaatgcagcgttcaaactgcttcctgaacatgatgtcgcttacgatggcaacccgctggcgcagcaacacggtccacgttacttca
tgctcaataagcctcagggctatgtttgctccacggacgaccctgatcacccaacggtgctctattttcttgatgaaccggtagcgtggaaactgc
atgcggcggggcggttggatattgataccaccggtctggtgctgatgactgatgatggtcagtggtcgcaccgcattacttctccgcgccatcatt
gcgagaagacctatctggtgacactggaatcacctgtagctgacgatacggcagagcaatttgctaaaggcgtgcagctgcataacgaaaaa
gatctcactaagcctgcggtgctggaagtgattaccccaacgcaggttcgtctgaccatcagcgaagggcgttatcatcaggtgaaacgcatgt
tcgccgccgtgggtaaccacgtggttgagctgcatcgtgaacgtattggcggtattacgctggatgctgatttagcccccggtgaatatcgtccgtt
aactgaagaagaaattgccagcgtcgtctaa (SEQ ID NO: 20)

## Figure 1 continued

6) yrfI
atgattatgccgcaacatgaccaattacatcgctatctgtttgaaaactttgccgtgcgcggcgaactggtaaccgtttcggaaaccctgcaacag
atccttgagaaccacgattatccgcagcccgttaaaaacgtgctggcagaactgctggttgcgaccagcctgttaaccgctacgctgaagtttga
tggtgatatcaccgtacagctgcagggcgacggtccgatgaatctggcggttattaacggtaacaataaccagcagatgcgcggtgtggcgcg
cgtgcagggcgaaattccagaaaatgccgacctgaaaacgctggtcggcaatggttacgtggtgatcaccattaccccgagcgaaggcgaa
cgctatcagggcgtagttggtctggaaggtgataccctggcggcctgcctggaagattactttatgcgttctgaacagctgccgacgcgcctgttt
attcgcaccggcgacgtagacggcaaaccggctgcaggcggtatgttgttgcaggtaatgcctgcgcaaaatgcccagcaggacgactttga
ccacctggcgacgctaaccgaaaccatcaaaaccgaagaactgctgaccttaccggcaaacgaagtgttgtggcgtttgtatcacgaagaag
aggtgacggtttacgatccgcaggatgtggagttcaaatgcacctgctcgcgtgaacgttgcgccgatgcgctgaaaacgctgcctgatgaag
aagttgatagcatcctggcggaagatggcgaaattgacatgcattgtgattactgcggtaaccactatctgttcaatgcgatggatattgctgaaat
ccgcaacaacgcgtctccggcagatccgcaagttcattaa (SEQ ID NO: 21)

7) yggJ
gtggggagacgacgcggatttttaactatgcgtatcccccgcatttatcatcctgaaccactgaccagccattctcacatcgcgctttgcgaagat
gccgccaaccatatcgggcgcgtactgcgcatggggccggggcaggcgttgcaattgtttgacggtagcaaccaggtctttgacgccgaaatt
accagcgccagcaaaaaaagcgtggaagtgaaggtgctggaaggccagatcgacgatcgcgaatctccgctgcatattcacctcggtcag
gtgatgtcgcgtggtgaaaaaatggaatttactatccagaaatcgatcgaactcggtgtaagcctcattacgccactttttctgagcgctgcggcg
ttaaactggatagtgaacgtctgaacaagaagcttcagcagtggcagaagattgcaattgctgcctgtgagcagtgtggtcgtaaccgggtgcc
ggaaatccgtccagcgatggatctggaagcctggtgtgcagagcaggatgaaggactgaaactgaatcttcacccgcgcgccagtaacagc
atcaatacgttgccgttaccggttgaacgcgtccgcctgctgattggcccggaaggcggtttatcggcagatgaaattgccatgactgcccgctat
caatttactgatatcctgttgggacctcgcgttttgcgtacagagacaactgcgctcaccgccattaccgcgctacaagtacgatttggcgatttgg
gctaa (SEQ ID NO: 22)

8) yjeE
atgatgaatcgagtaattccgctccctgatgagcaggcaacattagacctgggcgagcgggtagcgaaagcctgcgatggcgcaaccgtaat
ctatctgtatggcgatttaggcgcaggtaaaaccacctttagccggggctttttacaggctctgggtcatcagggtaatgtcaaaagccccacttat
acgctggtcgaaccctatacgctcgacaacttaatggtctatcactttgatttgtaccgccttgccgatcccgaggagctggagtttatggggatcc
gcgattattttgccaacgatgccatctgcctggtggagtggccacaacaaggtacaggtgttcttcctgacccggatgtcgaaatacacattgatt
atcaggcacaaggccgtgaggcgcgcgtgagtgcggtttcctctgcgggtgaattgttgctggcgcgtttagccggttaa
(SEQ ID NO: 23)

9) yiaO
atgaaattacgctctgtaacctacgcattattcattgctggcctggctgcattcagcacatcttctctggcggcacaatctttacgtttcggttatgaaa
catcacaaaccgactcgcaacatattgcggcgaaaaaattcaatgatttattgcaggagagaaccaaaggcgagctgaaattaaaactgttcc
cggacagcactctcggtaacgcgcaggcgatgatcagcggcgtacgtggcggcaccatcgatatggaaatgtccggctcgaataactttgcc
gggttatcaccagtgatgaacttgcttgatgtccctttcctgttccgcgataccgctcacgcgcataaaacgctcgacggcaaagtcggtgatgat
ctgaaagcctcacttgaaggtaaaggactgaaagtactggcctactgggaaaacggctggcgcgatgtcaccaactcgcgcgcaccggtta
aaacccccgccgacctgaaagggctgaaaatccgcaccaacaatagcccgatgaatatcgccgcattcaaagtctttggcgctaacccgatc
ccgatgccgtttgccgaagtctataccgggctggaaacccgcactatcgacgctcaggaacacccgatcaacgtcgtctggtcagcaaaatttt
tcgaagtgcagaagttcctttctctgacgcaccacgcctattccccgcttctggtggtgatcaacaaagcgaagtttgatggcttaagtccggagtt
ccagcaggcgctagtttcatctgcacaagaagcgggtaactatcagcgcaaactggttgctgaagatcagcaaaaaatcatcgacggcatga
aagaagcgggcgtggaagtcatcaccgatctcgaccgcaaagcctttagcgacgcactggggaatcaggttcgcgacatgtttgttaaagatg
tgccgcagggagctgatctgctgaaagccgtggatgaggtgcaataa (SEQ ID NO: 24)

10) yrdC
gtgaataataacctgcaaagagacgctatcgcagctgcgatagatgttctcaatgaagaacgtgtcatcgcctatccaacggaagccgttttcg
gtgttgggtgcgatcctgatagcgaaacagcagtgatgcgactgttggagttaaaacagcgtccggttgataaggggctgattttaatcgcagca
aattacgagcagcttaaaccctatattgatgacaccatgttgactgacgtgcagcgtgaaaccatttttcccgctggccaggtcctgtcacctttgt
ctttcccgcgcctgcgacaacaccgcgctggttgacgggccgctttgattcgcttgctgtacgagtcaccgaccatccgttggtggttgctttgtgcc
aggcttatggtaaaccgctggtttctaccagtgccaacttgagtggattgccaccttgtcgaacagtagacgaagttcgcgcacaatttggcgcg
gcgttcccggttgtgcctggtgaaacgggggggcgtttaaatccttcagaaatccgcgatgccctgacgggtgaactgtttcgacaggggtaa
(SEQ ID NO: 25)

## Figure 1 continued

11) yhbC
gtgggcttgtccacattagagcaaaaattaacagagatgattactgcgccagttgaggccctgggttttgaactggttggcatcgaatttattcgcg
gtcgcacatccacactgcgcatctatattgatagtgaagatggcatcaatgttgatgattgtgctgatgtgagccaccaggtaagtgctgtgctgg
atgttgaagatcccatcaccgttgcttataacctggaagtctcctcaccgggtctcgatcgcccactgttcacggctgaacactacgcccgttttgtc
ggagaagaggtgactctggttctccgtatggcggtacaaaaccgtcgtaaatggcagggcgttatcaaagcggtagacggtgaaatgatcac
agttaccgtcgaaggtaaagatgaagtgttcgcgctgagtaatatccagaaggcgaacctggttccccacttttaa (SEQ ID NO: 26)

12) ygbP
atggcaaccactcatttggatgtttgcgccgtggttccggcggccggatttggccgtcgaatgcaaacggaatgtcctaagcaatatctctcaatc
ggtaatcaaaccattcttgaacactcggtgcatgcgctgctggcgcatccccgggtgaaacgtgtcgtcattgccataagtcctggcgatagccg
ttttgcacaacttcctctggcgaatcatccgcaaatcaccgttgtagatggcggtgatgagcgtgccgattccgtgctggcaggtctgaaagccgc
tggcgacgcgcagtgggtattggtgcatgacgccgctcgtccttgtttgcatcaggatgacctcgcgcgattgttggcgttgagcgaaaccagcc
gcacggggggatcctcgccgcaccagtgcgcgatactatgaaacgtgccgaaccgggcaaaaatgccattgctcataccgttgatcgcaa
cggcttatggcacgcgctgacgccgcaatttttccctcgtgagctgttacatgactgtctgacgcgcgctctaaatgaaggcgcgactattaccga
cgaagcctcggcgctggaatattgcggattccatcctcagttggtcgaaggccgtgcggataacattaaagtcacgcgcccggaagatttggc
actggccgagttttacctcacccgaaccatccatcaggagaatacataa (SEQ ID NO: 27)

13) ybeY
atgagtcaggtgatcctcgatttacaactggcatgtgaagataattccgggttaccggaagagagccagtttcagacatggctgaatgcggtgat
cccgcagtttcaggaagaatcggaagtgacgattcgcgtggtcgataccgccgaaagccacagtctgaatctgacctatcgcggtaaggata
agccgaccaacgtgctctccttcccgtttgaagtgccgcctggcatggaaatgtcgctactgggcgatctggttatctgccgtcaggtggttgaga
aggaagctcaggagcaaggcaaaccactggaggcgcactgggcgcatatggtggtgcacggcagtctgcatttgttaggttacgatcacatc
gaagatgacgaagcagaagaaatggaagccctcgaaacagagattatgcttgctctgggctatgaggatccgtacattgccgagaaagaat
aa (SEQ ID NO: 28)

14) gcpE
atgcataaccaggctccaattcaacgtagaaaatcaacacgtatttacgttgggaatgtgccgattggcgatggtgctcccatcgccgtacagtc
catgaccaatacgcgtacgacagacgtcgaagcaacggtcaatcaaatcaaggcgctggaacgcgttggcgctgatatcgtccgtgtatccgt
accgacgatggacgcggcagaagcgttcaaactcatcaaacagcaggttaacgtgccgctggtggctgacatccacttcgactatcgcattgc
gctgaaagtagcggaatacggcgtcgattgtctgcgtattaaccctggcaatatcggtaatgaagagcgtattcgcatggtggttgactgtgcgc
gcgataaaaacattccgatccgtattggcgttaacgccggatcgctggaaaaagatctgcaagaaaagtatggcgaaccgacgccgcaggc
gttgctggaatctgccatgcgtcatgttgatcatctcgatcgcctgaacttcgatcagttcaaagtcagcgtgaaagcgtctgacgtcttcctcgctgt
tgagtcttatcgtttgctggcaaaacagatcgatcagccgttgcatctggggatcaccgaagccggtggtgcgcgcagcggggcagtaaaatc
cgccattggtttaggtctgctgctgtctgaaggcatcggcgacacgctgcgcgtatcgctggcggccgatccggtcgaagagatcaaagtcggtt
tcgatattttgaaatcgctgcgtatccgttcgcgagggatcaacttcatcgcctgcccgacctgttcgcgtcaggaatttgatgttatcggtacggtta
acgcgctggagcaacgcctggaagatatcatcactccgatggacgtttcgattatcggctgcgtggtgaatggcccaggtgaggcgctggtttct
acactcggcgtcaccggcggcaacaagaaaagcggcctctatgaagatggcgtgcgcaaagaccgtctggacaacaacgatatgatcgac
cagctggaagcacgcattcgtgcgaaagccagtcagctggacgaagcgcgtcgaattgacgttcagcaggttgaaaaataa
(SEQ ID NO: 29)

15) kdtB
atgcaaaaacgggcgatttatccgggtactttcgatcccattaccaatggtcatatcgatatcgtgacgcgcgccacgcagatgttcgatcacgtt
attctggcgattgccgccagccccagtaaaaaaccgatgtttaccctggaagagcgtgtggcactggcacagcaggcaaccgcgcatctggg
gaacgtggaagtggtcgggtttagtgatttaatggcgaacttcgcccgtaatcaacacgctacggtgctgattcgtggcctgcgtgcggtggcag
attttgaatatgaaatgcagctggcgcatatgaatcgccacttaatgccggaactggaaagtgtgtttctgatgccgtcgaaagagtggtcgtttat
ctcttcatcgttggtgaaagaggtggcgcgccatcagggcgatgtcacccatttcctgccggagaatgtccatcaggcgctgatggcgaagtta
gcgtag (SEQ ID NO: 30)

## Figure 1 continued

16) pfs
atgaaaatcggcatcattggtgcaatggaagaagaagttacgctgctgcgtgacaaaatcgaaaaccgtcaaactatcagtctcggcggttgc
gaaatctataccggccaactgaatggaaccgaggttgcgcttctgaaatcgggcatcggtaaagtcgctgcggcgctgggtgccactttgctgtt
ggaacactgcaagccagatgtgattattaacaccggttctgccggtggcctggcaccaacgttgaaagtgggcgatatcgttgtctcggacgaa
gcacgttatcacgacgcggatgtcacggcatttggttatgaatacggtcagttaccaggctgtccggcaggctttaaagctgacgataaactgat
cgctgccgctgaggcctgcattgccgaactgaatcttaacgctgtacgtggcctgattgttagcggcgacgctttcatcaacggttctgttggtctgg
cgaaaatccgccacaacttcccacaggccattgctgtagagatggaagcgacggcaatcgcccatgtctgccacaatttcaacgtcccgtttgtt
gtcgtacgcgccatctccgacgtggccgatcaacagtctcatcttagcttcgatgagttcctggctgttgccgctaaacagtccagcctgatggttg
agtcactggtgcagaaacttgcacatggctaa (SEQ ID NO: 31)

17) ycaJ
gtgagcaatctgtcgctcgatttttcggataatacttttcaacctctggccgcgcgtatgcggccagaaaatttagcacagtatatcggccagcaac
atttgctggctgcggggaagccgttgccgcgcgctatcgaagccgggcatttacattctatgatcctctgggggccgccgggtaccggcaaaac
aactctcgctgaagtgattgcccgctatgcgaacgctgatgtggaacgtatttctgccgtcacctctggcgtgaaagagattcgcgaggcgatcg
agcgcgcccggcaaaaccgcaatgcaggtcgccgcactattctttttgttgacgaagttcaccgtttcaacaaaagccagcaggatgcatttctg
ccacatattgaagacggcaccatcacttttattggcgcaaccactgaaaacccgtcgtttgagcttaattcggcactgctttcccgtgcccgtgtcta
tctgttgaaatccctgagtacagaggatattgagcaagtactaactcaggcgatggaagacaaaacccgtggctatggtggtcaggatattgttc
tgccagatgaaacacgacgcgccattgctgaactggtgaatggcgacgcgcgccgggcgttaaatacgctggaaatgatggcggatatggc
cgaagtcgatgatagcggtaagcgggtcctgaagcctgaattactgaccgaaatcgccggtgaacgtagcgcccgctttgataacaaaggcg
atcgcttttacgatctgatttccgcactgcataagtcggtacgtggtagcgcacccgatgcggcgctgtactggtatgcgcgaattattaccgctggt
ggcgatccgttatatgtcgcgcgtcgctgtctggcgattgcgtctgaagacgtcggtaatgccgatccacgggcgatgcaggtggcaattgcgg
cctgggattgctttactcgcgttggcccggcggaaggtgaacgcgccattgctcaggcgattgtttacctggcctgcgcgccaaaaagcaacgc
tgtctacactgcgtttaaagccgcgctggccgatgctcgcgaacgcccggattatgacgtgccggttcatttgcgtaatgcgccgacgaaattaat
gaaggaaatgggctacgggcaggaatatcgttacgctcatgatgaagcaaacgcttatgctgccggtgaggtttacttcccgccggaaatagc
acaaacacgctattatttcccgacaaacaggggccttgaaggcaagattggcgaaaagctcgcctggctggctgaacaggatcaaaatagc
cccataaaacgctaccgttaa (SEQ ID NO: 32)

18) b1808
gtgacggacgattttgcaccagacggtcagctggcgaaagcgataccaggctttaagccgcgagaaccacagcgacagatggcggtagcc
gtcacccaggcgatagaaaaaggccagccgctggtggtggaagcaggaaccggtacgggcaaaacctacgcttacctggctcctgcgctg
cgggcgaaaaagaaagtcattatctcgaccggctcaaaagcgttgcaggatcagctctacagccgcgatttgccaacagtctcaaaggcatt
gaaatatacgggcaacgtggcgctgctgaaagggcgctcaaactacctctgcctcgaacgtctcgaacagcaggcgctggcgggggcgat
ctgccggtacaaatcttaagcgatgtgatcctgctgcgctcctggtctaatcaaacagtcgatggtgatatcagcacctgcgtcagcgtggcgga
agattcacaggcgtggccgctggtcaccagcaccaacgacaactgtcttggcagcgactgcccgatgtataaagattgctttgtggtcaaagca
cgtaaaaaagcgatggacgccgatgtggtggtggtaaaccatcatctctttctggcggatatggtggttaaagagagtggatttggcgaactgat
cccggaagcggacgtcatgatcttcgacgaagcccaccagctaccggacattgccagccagtattttggtcagtcactctccagtcgacaactg
ctcgacctggcaaaagacatcaccatcgcctaccgcaccgaattaaaagacacccagcagttacaaaagtgcgctgatcgtcttgcccagag
tgcgcaggattttcgtctgcaactcggtgagccaggttatcgcggtaacctgcgtgagctgttagctaatccgcaaattcagcgggcatttttactg
ctcgatgacaccctggaactttgttatgacgtggcgaaactgtcactggggcgttccgccttgctggatgcggcatttgagcgcgccacgttgtatc
gcacacggctgaagcggctaaaagagatcaatcagccgggctacagctactggtacgaatgcacttcgcgccattttactctggctctcacgc
cgctcagcgtggcggataaattcaaagagttaatggcgcaaaaacccggtagctggatcttcacctcagcaacgctgtcggtgaacgacgat
ctgcatcatttcacctcgcggcttggcatcgaacaggccgagtcgttgctgttgcccagcccatttgattacagccgccaggcgttactctgtgtgct
gcgcaatctgccgcaaaccaaccagccaggttctgctcgccagttagcggcaatgctgcgaccgatcatcgaagctaacaacggtcgttgtttt
atgctttgtacctcgcacgccatgatgcgcgatctggccgagcagttccgcgctaccatgacgcttcctgtattgttgcaggggaaaccagcaa
agggcaactgttgcagcaatttgtcagcgccggtaatgcgcttcttgtggcaaccagcagtttctgggaaggggtggacgtgcgtggcgatacat
tgtcattggtaattatcgacaaattgccgtttacctcgccggatgatccactgttaaaagcgcgcatggaagattgtcgtttgcgcggtggcgaccc
gttcgatgaagtgcaactaccagatgccgtcattactctcaaacagggggtagggcgactgattcgcgacgccgacgatcgtggcgtgctggt
gatttgtgacaatcggctggtgatgcgtccttacggcgcgacgtttctcgccagtctgccgcccgcgccacgcacccgtgacattgcccgtgcgg
ttcgtttccttgcgataccatcctccaggtaa (SEQ ID NO: 33)

19) yeaA
atggctaataaaccttcggcagaagaactgaaaaaaaatttgtccgagatgcagttttacgtgacgcagaatcatgggacagaaccgccattt
acgggtcgtttactgcataacaagcgtgacggcgtatatcactgtttgatctgcgatgccccgctgtttcattcccaaaccaagtatgattccggctg
tggctggcccagtttctacgaaccggtaagtgaagaatccattcgttatatcaaagacttgtcacatggaatgcagcgcatagaaattcgttgcgg
taactgtgatgcccatctggggcatgtcttccccgacgggccgcagccaacgggcgaacgttattgtgttaactctgcctctttacgctttaccgatg
gcgaaaacggcgaagaaatcaacggttga (SEQ ID NO: 34)

Figure 1 continued

20) yagF
atgaccattgagaaaattttcaccccgcaggacgacgcgttttatgcggtgatcacccacgcggcggggccgcagggcgctctgccgctgacc
ccgcagatgctgatggaatctcccagcggcaacctgttcggcatgacgcagaacgccgggatgggctgggacgccaacaagctcaccggc
aaagaggtgctgattatcggcactcagggcggcatccgcgccggagacggacgcccaatcgcgctgggctaccacaccgggcattgggag
atcggcatgcagatgcaggcggcggcgaaggagatcacccgcaatggcgggatcccgttcgcggccttcgtcagcgatccgtgcgacggg
cgctcgcagggcacgcacggtatgttcgattccctgccgtaccgcaacgacgcggcgatcgtgtttcgccgcctgatccgctccctgccgacgc
ggcgggcggtgatcggcgtagcgacctgcgataaagggctgcccgccaccatgattgcgctggccgcgatgcacgacctgccgactattctg
gtgccgggcggggcgacgctgccgccgaccgtcggggaagacgcgggcaaggtgcagaccatcggcgcgcgtttcgccaaccacgaact
ctccctgcaggaggccgccgaactgggctgtcgcgcctgcgcctcgccgggcggcgggtgtcagttcctcggcacggcgggcacctcgcag
gtggtcgcggaggcgctgggtctggcgctgccgcactccgcgctggcgccgtccgggcaggcggtgtggctggagatcgcccgccagtcgg
cgcgcgcggtcagcgagctggatagccgcggcatcaccacgcgggatatcctctccgataaagccatcgaaaacgcgatggtgatccacgc
ggcgttcggcggctccaccaatttactgctgcacattccggccatcgcccacgcggcgggctgcacgatcccggacgttgagcactggacgcg
catcaaccgtaaagtgccgcgtctggtgagcgtgctgcccaacggcccggactatcacccgaccgtgcgcgccttcctcgcgggcggcgtgc
cggaggtgatgctccacctgcgcgacctcggcctgctgcatctggacgccatgaccgtgaccggccagacggtgggcgagaaccttgaatg
gtggcaggcgtccgagcgccgggcgcgcttccgccagtgcctgcgcgagcaggacggcgtagagccggatgacgtgatcctgccgccgga
gaaggcaaaagcgaaagggctgacctcgacggtctgcttcccgacgggcaacatcgctccggaaggttcggtgatcaaggccacggcgat
cgacccgtcggtggtgggcgaagatggcgtataccaccacaccggccgggtgcgggtgtttgtctcggaagcgcaggcgatcaaggcgatc
aagcgggaagagattgtgcagggcgatatcatggtggtgatcggcggcgggccgtccggcaccggcatggaagagacctaccagctcacc
tccgcgctaaagcatatctcgtggggcaagacggtgtcgctcatcaccgatgcgcgcttctcgggcgtgtcgacgggcgcctgcttcggccacg
tgtcgccggaggcgctggcgggcgggccgattggcaagctgcgcgataacgacatcatcgagattgccgtggatcgtctgacgttaactggc
agcgtgaacttcatcggcaccgcggacaacccgctgacgccggaagagggcgcgcgcgagctggcgcggcggcagacgcacccggac
ctgcacgcccacgacttttgccggacgacacccggctgtgggcggcactgcagtcggtgagcggcggcacctggaaaggctgtatttatgac
accgataaaattatcgaggtaattaacgccggtaaaaagcgctcggaatttaa (SEQ ID NO: 35)

21) b1983
gtgggacgtaaatgggccaatattgttgctaaaaaaacggctaaagacggtgcaacgtctaaaatttatgcaaaattcggtgtagaaatctatg
ctgctgctaaacaaggtgaacccgatccagaattaaacacatctttaaaattcgttattgaacgtgcaaagcaggcacaagttccaaagcacgt
tattgataaagcaattgataaagccaaaggcggcggagatgaaacgttcgtgcagggacgttatgaaggctttggtcctaatggctcaatgatt
atcgccgagacattgacttcaaatgttaaccgtacgattgctaacgttcgcacaattttcaataaaaaaggcggcaatatcggagcggcaggttc
tgtcagctatatgtttgacaatacgggtgtgattgtatttaaagggacagaccctgaccatattttgaaattttacttgaagctgaagttgatgttcgtg
atgtgactgaagaagaaggtaacattgttatttatactgaacctactgaccttcataaaggaatcgcggctctaaaagcagctggaatcactgag
ttctcaacaacagaattagaaatgattgctcaatctgaagttgagctttccccagaagatttagaaatctttgaagggcttgttgatgcccttgaaga
tgacgacgatgtacaaaaagtttatcataacgtcgcaaatctctaa (SEQ ID NO: 36)

22) yidD
atggcgccgccactgtcgcctggctcgcgggtcctgatagccctcattcgggtctatcaacgcctgattagtccgctactcggccgcattgtcgtt
tcactccaacctgttcaagctacggaattgaggcattgcgcaggtttggagtgataaaggcagttggttgacggtgaaacgcgtattaaaatgc
cacccttttacaccctggtggtgacgatcccgtcccgcccggaccatttgataccagagaacactaa (SEQ ID NO: 37)

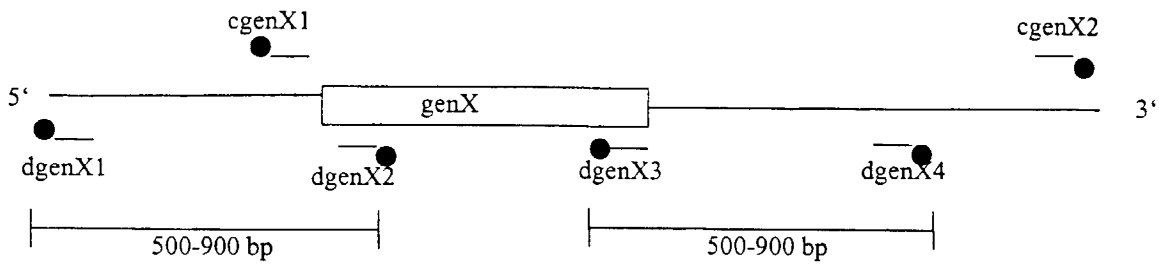
cgenX1
cgenX2
5'
genX
3'
dgenX1
dgenX2
dgenX3
dgenX4
500-900 bp
500-900 bp

Figure 2

Fig.3

| *E. coli* | | *B. subtilis* | | | | *H. influenzae* | | | *H. pylori* | | |
|---|---|---|---|---|---|---|---|---|---|---|---|
| gene name | GenBank#[1] | gene name | SWP#[2] or SubtiList#[3] | score | E-value | GenBank#[1] | score | E-value | GenBank#[1] | score | E-value |
| YgbB | g1789103 | YacN | [2]Q06756 | 169 | 2,00E-43 | 1573672 | 205 | 1,00E-54 | 2314164 | 105 | 1,00E-24 |
| YfhC | g1788911 | YaaJ | [2]P21335 | 135 | 2,00E-33 | 1573925 | 175 | 1,00E-45 | 2313814 | 24 | 3 |
| YacE | g1786292 | YtaG | [3]BG13824 | 135 | 2,00E-33 | 1573909 | 191 | 2,00E-50 | 2313965 | 87 | 4,00E-19 |
| YchB | g1787459 | YabH | [2]P37550 | 102 | 3,00E-23 | 1574450 | 317 | 3,00E-88 | 2314615 | 41 | 5,00E-05 |
| YejD | g1788510 | YtzF | [3]BG13940 | 126 | 6,00E-32 | 1574175 | 276 | 2,00E-74 | 2314637 | 61 | 5,00E-11 |
| Yrfl | g1789804 | YacC | [2]P37565 | 71 | 1,00E-13 | 1573822 | 286 | 5,00E-79 | 2314107 | 24 | 5,4 |
| YggJ | g1789315 | YqeU | [2]P54461 | 96 | 2,00E-21 | 1573272 | 312 | 1,00E-86 | 2313478 | 49 | 1,00E-07 |
| YjeE | g1790610 | YdiB | [3]BG12199 | 89 | 3,00E-19 | 1573014 | 171 | 1,00E-44 | 2313840 | 46 | 9,00E-07 |
| YiaO | g1790004 | YdbE | [3]BG12072 | 148 | 7,00E-37 | 1574060 | 374 | 1,00E-105 | 2314105 | 26 | 1,5 |
| YrdC | g2367210 | YwlC | [2]P39153 | 93 | 3,00E-19 | 1573655 | 206 | 3,00E-55 | 2313122 | 29 | 0,13 |
| YhbC | g1789561 | YlxS | [2]P32726 | 90 | 1,00E-19 | 1574740 | 157 | 2,00E-40 | 2314193 | 65 | 1,00E-12 |
| YgbP | g1789104 | YacM | [2]Q06755 | 129 | 3,00E-31 | 1573673 | 233 | 4,00E-63 | 2314164 | 47 | 5,00E-07 |
| YbeY | g1786880 | YqfG | [2]P46347 | 70 | 8,00E-14 | 1572948 | 190 | 2,00E-50 | 2314318 | 54 | 3,00E-09 |
| GcpE | g1788863 | YqfY | [2]P54482 | 318 | 4,00E-88 | 1573337 | 604 | 1,00E-174 | 2313753 | 294 | 3,00E-81 |
| KdtB | g1790065 | Ylbl | [3]BG13361 | 145 | 2,00E-36 | 1573650 | 176 | 5,00E-46 | 2314651 | 170 | 3,00E-44 |
| pfs | g1786354 | YrrU | [3]BG13800 | 244 | 6,00E-66 | 1574146 | 254 | 2,00E-69 | 2313168 | 123 | 4,00E-30 |
| YcaJ | g1787119 | YrvN | [3]BG13808 | 275 | 5,00E-75 | 1574435 | 668 | 0 | 2314168 | 201 | 3,00E-53 |
| b1808 | g1788110 | DinG | [2]P54394 | 245 | 6,00E-66 | 1573357 | 768 | 0 | 2313340 | 33 | 0,025 |
| yeaA | g1788077 | YppQ | [2]P54155 | 136 | 2,00E-32 | 1574293 | 116 | 3,00E-28 | 2313314 | 125 | 6,00E-31 |
| yagF | g1786464 | IlvD | [2]P51785 | 180 | 4,00E-46 | 1573744 | 168 | 7,00E-43 | 2314249 | 103 | 2,00E-23 |
| b1983 | g1788294 | Yeel | [3]BG12824 | 279 | 1,00E-76 | 1573285 | 161 | 2,00E-41 | 2313249 | 108 | 1,00E-25 |
| YidD | g140861 | YtjA | [3]BG13865 | 90 | 6,00E-19 | 1176311 | 96 | 1,00E-20 | 2314625 | 40 | 8,00E-04 |

[1]http://www.ncbi.nlm.nih.gov/Entrez/protein.html

[2]http://www.expasy.ch/sprot/

[3]http://www.pasteur.fr/Bio/SubtiList.html

[4](http://www.ncbi.nlm.nih.gov/BLAST/unfinishedgenome.html)

Fig.3 cont

| *E. coli* | | *M. tuberculosis* | | | *Ch. trachomatis* | | | *B. burgdorferi* | | |
|---|---|---|---|---|---|---|---|---|---|---|
| gene name | GenBank#[1] | GenBank#[1] | score | E-value | GenBank#[1] | score | E-value | GenBank#[1] | score | E-value |
| YgbB | g1789103 | 1877312 | 78 | 1,00E-16 | 3328865 | 62 | 1,00E-12 | 2688040 | 23 | 4,60E+00 |
| YfhC | g1788911 | 2960176 | 119 | 1,00E-28 | 3329316 | 120 | 2,00E-29 | 2687969 | 23 | 5,20E+00 |
| YacE | g1786292 | 2113915 | 119 | 1,00E-28 | 3328928 | 62 | 1,00E-11 | 2688463 | 41 | 2,00E-05 |
| YchB | g1787459 | 2052148 | 84 | 1,00E-17 | 3329270 | 82 | 2,00E-17 | 2688545 | 23 | 6,80E+00 |
| YejD | g1788510 | 2326754 | 109 | 2,00E-25 | 3329180 | 105 | 8,00E-25 | 2688006 | 90 | 5,00E-20 |
| Yrfl | g1789804 | 1550650 | 25 | 6,40E+00 | 3329168 | 23 | 7,80E+00 | 2688577 | 23 | 5,40E+00 |
| YggJ | g1789315 | 2078027 | 70 | 2,00E-13 | 3328922 | 23 | 8,60E+00 | 2688252 | 44 | 4,00E-06 |
| YjeE | g1790610 | 1449365 | 60 | 1,00E-10 | 3328975 | 61 | 2,00E-11 | 2688077 | 68 | 1,00E-13 |
| YiaO | g1790004 | 2113942 | 27 | 1,80E+00 | 3328868 | 26 | 1,30E+00 | 2688570 | 25 | 2,70E+00 |
| YrdC | g2367210 | 1322425 | 68 | 8,00E-13 | 3328537 | 68 | 2,00E-13 | 2688669 | 73 | 3,00E-15 |
| YhbC | g1789561 | 2078017 | 56 | 2,00E-09 | 3328787 | 26 | 4,00E-01 | 2688749 | 38 | 1,00E-04 |
| YgbP | g1789104 | 1877313 | 99 | 3,00E-22 | 3328896 | 95 | 2,00E-21 | 2688781 | 26 | 6,30E-01 |
| YbeY | g1786880 | 2078032 | 62 | 3,00E-11 | 3328852 | 26 | 4,20E-01 | 2687941 | 55 | 6,00E-10 |
| GcpE | g1788863 | 2612813 | 277 | 9,00E-76 | 3328450 | 155 | 2,00E-39 | 2688019 | 31 | 5,30E-02 |
| KdtB | g1790065 | 1694866 | 140 | 8,00E-35 | 3329163 | 25 | 9,40E-01 | 2688628 | 97 | 2,00E-22 |
| pfs | g1786354 | 1405762 | 100 | 1,00E-22 | 3328855 | 22 | 6,80E-01 | 2688288 | 152 | 8,00E-39 |
| YcaJ | g1787119 | 1460081 | 329 | 3,00E-91 | 3328753 | 60 | 1,00E-10 | 2688379 | 55 | 4,00E-09 |
| b1808 | g1788110 | 1340095 | 274 | 1,00E-74 | 3329029 | 27 | 9,30E-01 | 2688551 | 30 | 1,30E-01 |
| yeaA | g1788077 | 1550715 | 126 | 8,00E-31 | 3328854 | 27 | 2,20E-01 | 2688358 | 23 | 3,80E+00 |
| yagF | g1786464 | 2213526 | 195 | 1,00E-50 | 3329033 | 28 | 7,30E-01 | 2688576 | 25 | 4,40E+00 |
| b1983 | g1788294 | 2281051 | 124 | 8,00E-30 | 3328890 | 118 | 1,00E-28 | 2687898 | 138 | 1,00E-34 |
| YidD | g140861 | 2808707 | 73 | 1,00E-13 | 3328908 | 56 | 2,00E-08 | 2688025 | 52 | 3,00E-07 |

Fig.3 cont.

| *E. coli* | | *T. pallidum* | | | *S. pneumoniae** | | | *S. aureus** | | |
|---|---|---|---|---|---|---|---|---|---|---|
| gene name | GenBank#[1] | GenBank#[1] | score | E-value | contig#[4] | score | E-value | contig#[4] | score | E-value |
| YgbB | g1789103 | 3322804 | 98 | 1,00E-22 | / | n.d. | n.d. | / | n.d. | n.d. |
| YfhC | g1788911 | 3322548 | 33 | 4,00E-03 | 101 | 71 | 2,00E-12 | 49 | 102 | 2,00E-25 |
| YacE | g1786292 | 3322572 | 36 | 6,00E-04 | 17 | 109 | 3,00E-24 | / | n.d. | n.d. |
| YchB | g1787459 | 3322649 | 83 | 7,00E-18 | / | n.d. | n.d. | / | n.d. | n.d. |
| YejD | g1788510 | 3322747 | 97 | 3,00E-22 | 41 | 166 | 2,00E-41 | 12 | 152 | 5,00E-37 |
| Yrfl | g1789804 | / | n.d. | n.d. | 7 | 80 | 2,00E-15 | 249 | 82 | 9,00E-16 |
| YggJ | g1789315 | 3322550 | 27 | 4,90E-01 | 93 | 65 | 1,00E-10 | 90 | 86 | 7,00E-17 |
| YjeE | g1790610 | 3323187 | 76 | 6,00E-16 | 140 | 80 | 2,00E-15 | 24 | 75 | 9,00E-14 |
| YiaO | g1790004 | 3322488 | 28 | 2,90E-01 | / | n.d. | n.d. | / | n.d. | n.d. |
| YrdC | g2367210 | 3322447 | 39 | 6,00E-05 | 123 | 62 | 9,00E-10 | 193 | 76 | 3,00E-14 |
| YhbC | g1789561 | 3322709 | 26 | 4,40E-01 | 47 | 55 | 8,00E-08 | 173 | 90 | 1,00E-18 |
| YgbP | g1789104 | 3322804 | 58 | 2,00E-10 | 72 | 55 | 2,00E-07 | / | n.d. | n.d. |
| YbeY | g1786880 | 3322948 | 48 | 1,00E-07 | 17 | 60 | 2,00E-09 | 396 | 75 | 7,00E-14 |
| GcpE | g1788863 | 3322731 | 217 | 3,00E-58 | / | n.d. | n.d. | / | n.d. | n.d. |
| KdtB | g1790065 | 3322553 | 100 | 2,00E-23 | 232 | 113 | 2,00E-25 | 205 | 149 | 2,00E-36 |
| pfs | g1786354 | 3322437 | 112 | 9,00E-27 | 156 | 182 | 5,00E-46 | 1235 | 82 | 1,00E-15 |
| YcaJ | g1787119 | 3323329 | 53 | 1,00E-08 | 62 | 95 | 2,00E-23 | 1085 | 159 | 1,00E-38 |
| b1808 | g1788110 | 3322379 | 29 | 3,50E-01 | 114 | 114 | 5,00E-25 | 434 | 34 | 7,40E-01 |
| yeaA | g1788077 | 3322932 | 111 | 1,00E-26 | 31 | 136 | 2,00E-33 | 422 | 112 | 1,00E-25 |
| yagF | g1786464 | 3322975 | 26 | 3,20E+00 | 38 | 202 | 1,00E-51 | 24 | 171 | 4,00E-42 |
| b1983 | g1788294 | 3322762 | 142 | 9,00E-36 | 143 | 360 | 1,00E-99 | 412 | 183 | 2,00E-46 |
| YidD | g140861 | Treponema_p | 71 | 4,00E-13 | 12 | 64 | 7,00E-11 | 1341 | 76 | 1,00E-14 |

Fig.3 cont.

| *E. coli* | | *E. faecalis** | | | *P.aeruginosa** | | | *B. pertussis** | | |
|---|---|---|---|---|---|---|---|---|---|---|
| gene name | GenBank#[1] | contig#[4] | score | E-value | contig#[4] | score | E-value | contig#[4] | score | E-value |
| YgbB | g1789103 | 6177 | 141 | 8,00E-34 | 93 | 181 | 5,00E-46 | 126 | 139 | 3,00E-33 |
| YfhC | g1788911 | 6349 | 132 | 3,00E-31 | 93 | 151 | 7,00E-37 | 737 | 151 | 9,00E-37 |
| YacE | g1786292 | 6196 | 111 | 1,00E-24 | 95 | 187 | 1,00E-47 | 924 | 159 | 3,00E-39 |
| YchB | g1787459 | 6342 | 114 | 2,00E-25 | 95 | 286 | 2,00E-77 | 1062 | 215 | 9,00E-56 |
| YejD | g1788510 | 6178 | 137 | 2,00E-32 | 94 | 198 | 8,00E-51 | 983 | 91 | 1,00E-18 |
| Yrfl | g1789804 | 6199 | 97 | 2,00E-20 | 97 | 192 | 4,00E-49 | 1085 | 160 | 2,00E-39 |
| YggJ | g1789315 | 6287 | 75 | 1,00E-13 | 66 | 196 | 4,00E-50 | 551 | 119 | 4,00E-27 |
| YjeE | g1790610 | 6294 | 29 | 4,60E+00 | 97 | 177 | 7,00E-45 | 762 | 125 | 4,00E-29 |
| YiaO | g1790004 | 6236 | 125 | 1,00E-28 | 91 | 139 | 8,00E-33 | 459 | 201 | 1,00E-51 |
| YrdC | g2367210 | 6288 | 96 | 4,00E-20 | 75 | 163 | 2,00E-40 | 362 | 43 | 4,00E-05 |
| YhbC | g1789561 | 6465 | 103 | 2,00E-22 | 85 | 148 | 6,00E-36 | 371 | 76 | 4,00E-14 |
| YgbP | g1789104 | 6311 | 55 | 2,00E-07 | 93 | 180 | 2,00E-45 | 126 | 93 | 5,00E-19 |
| YbeY | g1786880 | 6286 | 67 | 1,00E-11 | 91 | 142 | 3,00E-34 | 369 | 89 | 5,00E-18 |
| GcpE | g1788863 | / | n.d. | n.d. | 91 | 514 | 1,00E-145 | 862 | 161 | 2,00E-39 |
| KdtB | g1790065 | 6384 | 147 | 1,00E-35 | 84 | 197 | 1,00E-50 | 1097 | 172 | 2,00E-43 |
| pfs | g1786354 | 6495 | 201 | 1,00E-51 | / | n.d. | n.d. | / | n.d. | n.d. |
| YcaJ | g1787119 | 6287 | 138 | 2,00E-32 | 89 | 529 | 1,00E-150 | 1043 | 452 | 1,00E-127 |
| b1808 | g1788110 | 6265 | 120 | 7,00E-27 | 82 | 215 | 1,00E-55 | 781 | 255 | 1,00E-67 |
| yeaA | g1788077 | 6315 | 138 | 3,00E-33 | 81 | 158 | 2,00E-39 | 777 | 146 | 1,00E-35 |
| yagF | g1786464 | / | n.d. | n.d. | 84 | 169 | 1,00E-41 | 759 | 160 | 8,00E-39 |
| b1983 | g1788294 | 6169 | 309 | 3,00E-84 | 82 | 145 | 5,00E-35 | 1059 | 155 | 6,00E-38 |
| YidD | g140861 | / | n.d. | n.d. | 46 | 76 | 1,00E-14 | 1007 | 74 | 7,00E-14 |

European Patent Office

# PARTIAL EUROPEAN SEARCH REPORT

which under Rule 45 of the European Patent Convention shall be considered, for the purposes of subsequent proceedings, as the European search report

Application Number EP 99 10 7031

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 42875 A (UNIV CASE WESTERN RESERVE) 1 October 1998 (1998-10-01) * page 21, line 28 - page 22, line 13 * * page 27, line 6 - page 30, line 21 * * claims 7-11; figure 4; examples 2,4 * --- | 1-5,14, 17-19,21 | C12Q1/18 C07K14/245 C12Q1/68 |
| A | MOIR, D.T. ET AL.: "Genomics and Antimicrobial Drug Discovery" ANTIMICROBIAL AGENTS AND CHEMOTHERAPY, vol. 43, no. 3, March 1999 (1999-03), pages 439-446, XP000921053 * page 439, column 1, line 26 - column 2, line 5 * * page 440 - page 445, column 1 * --- -/-- | 1-6,14, 17-19,21 | |

TECHNICAL FIELDS SEARCHED (Int.Cl.7)

C12Q
G01N
C07K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 6 July 2000 | Gundlach, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

## INCOMPLETE SEARCH SHEET C

Application Number
EP 99 10 7031

Claim(s) not searched:
7-10

Reason for the limitation of the search:

Present claims 7-10 relate to a product defined by reference to a desirable characteristic or property, namely being "identified by the method of any one of claims 1 to 6".

The claims cover all products having this characteristic or property, whereas the application provides no support within the meaning of Article 84 EPC and/or disclosure within the meaning of Article 83 EPC for such products. In the present case, the claims so lack support, and the application so lacks disclosure, that a meaningful search over the claimed scope is impossible. Independent of the above reasoning, the claims also lack clarity (Article 84 EPC). An attempt is made to define the product by reference to a result to be achieved. Again, this lack of clarity in the present case is such as to render a meaningful search over the claimed scope impossible. Consequently, the search has not been carried out for these claims.

European Patent Office

# PARTIAL EUROPEAN SEARCH REPORT

Application Number
EP 99 10 7031

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | ARIGONI F ET AL: "A genome-based approach for the identification of essential bacterial genes" NATURE BIOTECHNOLOGY,US,NATURE PUBLISHING, vol. 16, no. 16, September 1998 (1998-09), pages 851-856-856, XP002124132 ISSN: 1087-0156 * abstract * * page 855, column 1, paragraph 4 - column 2, paragraph 1 * * page 856, column 2, line 57 - line 58 * --- | 1-6,14, 17-19,21 | |
| A | BALTZ ET AL: "DNA sequence sampling of the Streptococcus pneumoniae genome to identify novel targets for antibiotic development" MICROBIAL DRUG RESISTANCE,US,LIEBERT, vol. 4, no. 1, 21 March 1998 (1998-03-21), pages 1-9-9, XP002112153 ISSN: 1076-6294 * abstract; tables 1,4 * * page 8, column 1, paragraph 2 * --- | 1-6,14, 17-19,21 | TECHNICAL FIELDS SEARCHED (Int.Cl.7) |
| A | TRIAS AND GORDON: "INNOVATIVE APPROACHES TO NOVEL ANTIBACTERIAL DRUG DISCOVERY" CURRENT OPINION IN BIOTECHNOLOGY,GB,LONDON, vol. 8, no. 8, 1997, pages 757-762-762, XP002119418 ISSN: 0958-1669 * the whole document * --- | 1-6,14, 17-19,21 | |

-/--

EPO FORM 1503 03.82 (P04C10)

European Patent Office

# PARTIAL EUROPEAN SEARCH REPORT

Application Number
EP 99 10 7031

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | TATUSOV R L ET AL: "METABOLISM AND EVOLUTION OF HAEMOPHILUS INFLUENZAE DEDUCED FROM A WHOLE-GENOME COMPARISON WITH ESCHERICHIA COLI"<br>CURRENT BIOLOGY,GB,CURRENT SCIENCE,,<br>vol. 6, no. 3, 1 March 1996 (1996-03-01), pages 279-291-291, XP000857871<br>ISSN: 0960-9822<br>* the whole document *<br>--- | 1-6,14, 17-19,21 | |
| A | FLEISCHMANN R D ET AL: "WHOLE-GENOME RANDOM SEQUENCING AND ASSEMBLY OF HAEMOPHILUS INFLUENZAE RD"<br>SCIENCE,US,AMERICAN ASSOCIATION FOR THE ADVANCEMENT OF SCIENCE,,<br>vol. 269, no. 5223,<br>28 July 1995 (1995-07-28), pages 496-498,507-51, XP000517090<br>ISSN: 0036-8075<br>* the whole document *<br>--- | 1-6,14, 17-19,21 | TECHNICAL FIELDS SEARCHED (Int.Cl.7) |
| E | WO 99 47553 A (ARIGONI FABRIZIO ;PEITSCH MANUEL C (CH); LOFERER HANNES (DE); GLAX)<br>23 September 1999 (1999-09-23)<br>* the whole document *<br>--- | 1-6,14, 17-19,21 | |
| T | ROHDICH, F. ET AL.: "Cytidine 5'-triphosphate-dependent biosynthesis of isoprenoids: YgbP protein of Escherichia coli catalyzes the formation of 4-diphosphocytidyl-2-C-methylerythritol"<br>PNAS,<br>vol. 96, no. 21,<br>12 October 1999 (1999-10-12), pages 11758-11763, XP002132607<br>* abstract *<br>---<br>-/-- | 1-5,14, 17-19,21 | |

EPO FORM 1503 03.82 (P04C10)

European Patent Office

# PARTIAL EUROPEAN SEARCH REPORT

Application Number
EP 99 10 7031

| Category | Citation of document with indication, where appropriate, of relevant passages | Relevant to claim | CLASSIFICATION OF THE APPLICATION (Int.Cl.7) |
|---|---|---|---|
| A | POULSEN, L.K. ET AL.: "Analysis of an Escherichia coli mutant strain resistant to the cell-killing function encoded by the gef gene family" MOLECULAR MICROBIOLOGY, vol. 6, no. 7, 1992, pages 895-905, XP000923116 * the whole document * --- | 1-4,14, 17-19,21 | |
| A | FULLNER, K.J. ET AL.: "Genetic Characterization of a New Type IV-A Pilus Gene Cluster Found in Both Classical and El Tor Biotypes of Vibrio cholerae" INFECTION AND IMMUNITY, vol. 67, no. 3, March 1999 (1999-03), pages 1393-1404, XP000914871 * the whole document * --- | 1-4,14, 17-19,21 | TECHNICAL FIELDS SEARCHED (Int.Cl.7) |
| A | POST, D.A. ET AL.: "Characterization of the hemA-prs region of the Escherichia coli and Salmonella typhimurium chromosomes: identification of two open reading frames and implications for prs expression" JOURNAL OF GENERAL MICROBIOLOGY, vol. 139, no. 2, February 1993 (1993-02), pages 259-266, XP000923112 * abstract * * page 265, column 1, paragraph 4 * --- | 1-4,14, 17-19,21 | |
| A | BENTLEY, J. ET AL.: "Cloning and sequence analysis of an Escherichia coli gene conferring bicyclomycin resistance" GENE, vol. 127, 1993, pages 117-120, XP000914847 * the whole document * --- | 1-4,14, 17-19,21 | |
| | -/-- | | |

EPO FORM 1503 03.82 (P04C10)

European Patent Office

# PARTIAL EUROPEAN SEARCH REPORT

Application Number
EP 99 10 7031

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | TSUI, H.C.T ET AL.: "The mutL repair gene of Escherichia coli K-12 forms a superoperon with a gene encoding a new cell-wall amidase" MOLECULAR MICROBIOLOGY, vol. 11, no. 1, 1994, pages 189-202, XP000921221 * the whole document * --- | 1-4,14, 17-19,21 | |
| A | CONNOLLY, D.M. ET AL.: "Genetic and Physiological Relationships among the miaA Gene, 2-Methylthio-N6-(delta2-Isopentenyl)-Adenosine tRNA Modification, and Spontaneous Mutagenesis in Escherichia coli K-12" JOURNAL OF BACTERIOLOGY, vol. 171, no. 6, June 1989 (1989-06), pages 3233-3246, XP000921249 * the whole document * --- | 1-4,14, 17-19,21 | TECHNICAL FIELDS SEARCHED (Int.Cl.7) |
| T | ESBERG, B. ET AL.: "Identification of the miaB Gene, Involved in Methylthiolation of Isopentenylated A37 Derivatives in the tRNA of Salmonella typhimurium and Escherichia coli" JOURNAL OF BACTERIOLOGY, vol. 181, no. 23, December 1999 (1999-12), pages 7256-7265, XP000922660 * the whole document * --- -/-- | 1-4,14, 17-19,21 | |

EPO FORM 1503 03.82 (P04C10)

European Patent Office

# PARTIAL EUROPEAN SEARCH REPORT

Application Number
EP 99 10 7031

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | RATHER, P.N. ET AL.: "aarC, an Essential Gene Involved in Density-Dependent Regulation of the 2'-N-Acetyltransferase in Providencia stuartii" JOURNAL OF BACTERIOLOGY, vol. 179, no. 7, April 1997 (1997-04), pages 2267-2273, XP000914850 * abstract * * page 2272, column 1, paragraph 3 - column 2, paragraph 1 * --- | 1-4,14, 17-19,21 | |
| A | GUASCH, J.F. ET AL.: "Cloning and Characterization of Two Serratia marcescens Genes Involved in Core Lipopolysaccharide Biosynthesis" JORNAL OF BACTERIOLOGY, vol. 178, no. 19, October 1996 (1996-10), pages 5741-5747, XP000914851 * abstract * --- | 1-4,14, 17-19,21 | TECHNICAL FIELDS SEARCHED (Int.Cl.7) |
| E | WO 00 17387 A (PANTHERIX LIMITED ;SHAW WILLIAM VARE (GB); LEWENDON ANN (GB)) 30 March 2000 (2000-03-30) * the whole document * --- | 1-4,14, 17-19,21 | |
| A | CORNELL, K.A. ET AL.: "Cloning and expression of Escherichia coli 5'-methylthioadenosine/S-adenosylhomocysteine nucleosidase: Identification of the pfs gene product" BIOCHIMICA ET BIOPHYSICA ACTA, vol. 1396, 1998, pages 8-14, XP000921381 * the whole document * --- | 1-4,14, 17-19,21 | |
| X | WO 98 06734 A (KNOWLES DAVID JUSTIN CHARLES ;BLACK MICHAEL TERENCE (US); HODGSON) 19 February 1998 (1998-02-19) * claims 10,17 * --- | 1-4,14, 17-19,21 | |

-/--

EPO FORM 1503 03.82 (P04C10)

European Patent Office

# PARTIAL EUROPEAN SEARCH REPORT

Application Number
EP 99 10 7031

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | HANSEN, F.G. ET AL.: "Physical mapping and nucleotide sequence of the rnpA gene that encodes the protein component of ribonuclease P in Escherichia coli" GENE, vol. 38, 1985, pages 85-93, XP000915093 * the whole document * --- | 1-4,14, 17-19,21 | |
| A | SKOVGAARD, OLE: "Nucleotide sequence of a Proteus mirabilis DNA fragment homologous to the 60K-rnpA-rpmH-dnaA-dnaN-recF-gyrB region of Escherichia coli" GENE, vol. 93, 1990, pages 27-34, XP000914868 * the whole document * --- | 1-4,14, 17-19,21 | TECHNICAL FIELDS SEARCHED (Int.Cl.7) |
| A | WO 95 09925 A (ZENECA LTD ;HAWKES TIMOTHY ROBERT (GB)) 13 April 1995 (1995-04-13) * abstract; claims 1,8 * --- | 1-5,14, 17-19,21 | |
| A | WO 96 35804 A (MERCK & CO INC ;POMPLIANO DAVID L (US); BRAMHILL DAVID (US); CUNNI) 14 November 1996 (1996-11-14) * abstract; claims 1,5 * --- | 1-5,14, 17-19,21 | |
| A | WO 98 35054 A (RIBOGENE INC) 13 August 1998 (1998-08-13) * abstract; claims 1,4,8,29 * --- | 1-5,14, 17-19,21 | |
| A | WO 99 14311 A (SMITHKLINE BEECHAM CORP ;COLEMAN KENNETH (US); CRITCHLEY IAN A (US) 25 March 1999 (1999-03-25) * abstract; claims 1,3 * ----- | 1-5,14, 17-19,21 | |

EPO FORM 1503 03.82 (P04C10)

European Patent Office

Application Number

EP 99 10 7031

## CLAIMS INCURRING FEES

The present European patent application comprised at the time of filing more than ten claims.

- [X] Only part of the claims have been paid within the prescribed time limit. The present European search report has been drawn up for the first ten claims and for those claims for which claims fees have been paid, namely claim(s):

  14,17-19,21

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [X] Only part of the further search fees have been paid within the fixed time limit. The present European search report has been drawn up for those parts of the European patent application which relate to the inventions in respect of which search fees have been paid, namely claims:

  1-10,14,17-19,21 (partially)

- [ ] None of the further search fees have been paid within the fixed time limit. The present European search report has been drawn up for those parts of the European patent application which relate to the invention first mentioned in the claims, namely claims:

European Patent Office

**LACK OF UNITY OF INVENTION SHEET B**

Application Number
EP 99 10 7031

The Search Division considers that the present European patent application does not comply with the requirements of unity of invention and relates to several inventions or groups of inventions, namely:

1. Claims: 1-10,14,17-19,21 (partially)

   Method for identifying antimicrobial agents using ygbB or ygbP

2. Claims: 1-10,14,17-19,21 (partially)

   Method for identifying antimicrobial agents using yfhC

3. Claims: 1-10,14,17-19,21 (partially)

   Method for identifying antimicrobial agents using yacE

4. Claims: 1-10,14,17-19,21 (partially)

   Method for identifying antimicrobial agents using ychB

5. Claims: 1-10,14,17-19,21 (partially)

   Method for identifying antimicrobial agents using yejD

6. Claims: 1-10,14,17-19,21 (partially)

   Method for identifying antimicrobial agents using yrfI

7. Claims: 1-10,14,17-19,21 (partially)

   Method for identifying antimicrobial agents using yggJ

8. Claims: 1-10,14,17-19,21 (partially)

   Method for identifying antimicrobial agents using yjeE

9. Claims: 1-10,14,17-19,21 (partially)

   Method for identifying antimicrobial agents using yiaO

10. Claims: 1-10,14,17-19,21 (partially)

    Method for identifying antimicrobial agents using yrdC

11. Claims: 1-10,14,17-19,21 (partially)

European Patent Office

**LACK OF UNITY OF INVENTION SHEET B**

Application Number
EP 99 10 7031

The Search Division considers that the present European patent application does not comply with the requirements of unity of invention and relates to several inventions or groups of inventions, namely:

Method for identifying antimicrobial agents using yhbC

12. Claims: 1-10,14,17-19,21 (partially)

    Method for identifying antimicrobial agents using ybeY

13. Claims: 1-10,14,17-19,21 (partially)

    Method for identifying antimicrobial agents using gcpE

14. Claims: 1-10,14,17-19,21 (partially)

    Method for identifying antimicrobial agents using kdtB

15. Claims: 1-10,14,17-19,21 (partially)

    Method for identifying antimicrobial agents using pfs

16. Claims: 1-10,14,17-19,21 (partially)

    Method for identifying antimicrobial agents using ycaJ

17. Claims: 1-10,14,17-19,21 (partially)

    Method for identifying antimicrobial agents using b1808

18. Claims: 1-10,14,17-19,21 (partially)

    Method for identifying antimicrobial agents using yeaA

19. Claims: 1-10,14,17-19,21 (partially)

    Method for identifying antimicrobial agents using yagF

20. Claims: 1-10,14,17-19,21 (partially)

    Method for identifying antimicrobial agents using b1983

21. Claims: 1-10,14,17-19,21 (partially)

    Method for identifying antimicrobial agents using yidD

European Patent Office

**LACK OF UNITY OF INVENTION**
**SHEET B**

Application Number
EP 99 10 7031

The Search Division considers that the present European patent application does not comply with the requirements of unity of invention and relates to several inventions or groups of inventions, namely:

## ANNEX TO THE EUROPEAN SEARCH REPORT ON EUROPEAN PATENT APPLICATION NO. EP 99 10 7031

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2000

| Patent document cited in search report | Publication date | Patent family member(s) | Publication date |
|---|---|---|---|
| WO 9842875 A | 01-10-1998 | US 5858367 A | 12-01-1999 |
| | | AU 6589098 A | 20-10-1998 |
| | | EP 0975801 A | 02-02-2000 |
| WO 9947553 A | 23-09-1999 | AU 2947399 A | 11-10-1999 |
| WO 0017387 A | 30-03-2000 | AU 6102899 A | 10-04-2000 |
| WO 9806734 A | 19-02-1998 | CA 2237162 A | 17-01-1999 |
| | | EP 0956289 A | 17-11-1999 |
| | | EP 0893505 A | 27-01-1999 |
| | | US 5840560 A | 24-11-1998 |
| | | US 5940560 A | 17-08-1999 |
| | | US 5932701 A | 03-08-1999 |
| WO 9509925 A | 13-04-1995 | AU 686231 B | 05-02-1998 |
| | | AU 7702394 A | 01-05-1995 |
| | | EP 0722505 A | 24-07-1996 |
| | | HU 73691 A | 30-09-1996 |
| | | JP 9503131 T | 31-03-1997 |
| | | NZ 273643 A | 19-12-1997 |
| WO 9635804 A | 14-11-1996 | NONE | |
| WO 9835054 A | 13-08-1998 | AU 6155398 A | 26-08-1998 |
| | | US 5998159 A | 07-12-1999 |
| WO 9914311 A | 25-03-1999 | NONE | |

EPO FORM P0459

For more details about this annex : see Official Journal of the European Patent Office, No. 12/82